(19) Europäisches Patentamt / European Patent Office / Office européen des brevets

(11) **EP 2 444 396 A1**

(12) **EUROPEAN PATENT APPLICATION**
published in accordance with Art. 153(4) EPC

(43) Date of publication:
**25.04.2012 Bulletin 2012/17**

(21) Application number: **10789042.8**

(22) Date of filing: **15.06.2010**

(51) Int Cl.:
***C07D 233/64*** *(2006.01)* ***A61K 8/49*** *(2006.01)*
***A61Q 17/04*** *(2006.01)*

(86) International application number:
**PCT/ES2010/070397**

(87) International publication number:
**WO 2010/146210 (23.12.2010 Gazette 2010/51)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **16.06.2009 ES 200901426**

(71) Applicant: **Apoteknos Para La Piel, S.L.**
**28005 Madrid (ES)**

(72) Inventors:
- **PIVEL RANIERI, Juan Pablo**
  **E-28224 Pozuelo de Alarcón (Madrid) (ES)**
- **FERRER CUESTA, Juan Manuel**
  **E-28760 Tres Cantos (Madrid) (ES)**
- **FORT, Diego**
  **E-41092 Sevilla (ES)**
- **TABAREZ, Carlos**
  **Pando**
  **Canelones (UY)**
- **MOYNA, Patrick**
  **Montevideo**
  **UY-11400 (UY)**

(74) Representative: **ABG Patentes, S.L.**
**Avenida de Burgos 16D**
**Edificio Euromor**
**28036 Madrid (ES)**

(54) **PHOTOPROTECTIVE COMPOSITION**

(57) The present invention relates to a composition comprising (i) a compound A that absorbs in the near visible- ultraviolet A (UVA) region and (ii) a compound B that absorbs in the ultraviolet B (UVB) region, wherein at least one of said compounds A or B is a compound of formula (I) wherein Y represents an optionally substituted aryl or styryl-aryl group, $R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and $R^5$ and $R^6$ independently of each other represent H, or alternatively $R^5$ and $R^6$ together form a C-C bond between the carbon atoms to which they are bound. The invention also relates to the use of said compound of formula (I) in the preparation of a photoprotective composition and to the related cosmetic treatment.

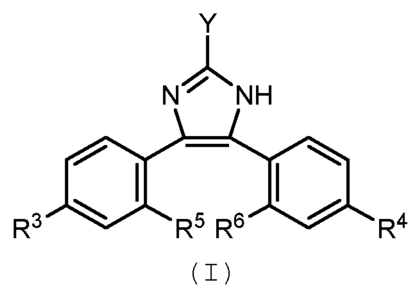

(I)

EP 2 444 396 A1

## Description

### Field of the Invention

The present invention relates to a photoprotective composition useful for preventing or minimizing the damaging effects produced by solar radiation on the skin or hair.

### Background of the Invention

Electromagnetic radiations emitted by the sun are directly responsible for the harmful effects of the sun on skin and hair. Their consequences at the skin level depend on multiple factors, mainly on the type of radiation (wavelength, exposure time and skin penetration level), the skin phototype and the individual susceptibility. Likewise, solar radiations that reach the Earth depend on different factors such as the latitude, the altitude above sea level, the season of the year, the time of the day, the thickness of the ozone layer, etc.

The region of the electromagnetic spectrum known as near visible ultraviolet can in turn be divided into four regions (UVC, UVB, UVA and near visible), the characteristics of which are described below.

The UVC rays (200-280 nm) emitted by the sun form the ultraviolet radiation with higher energy content. However, this radiation is not dangerous since the UVC solar rays are filtered through the ozone layer in the stratosphere, not reaching the Earth's surface.

UVB radiation (280-320 nm) with an intermediate energy level between UVC rays and UVA rays reaches the Earth's surface fairly filtered. This type of radiation penetrates the skin to a small extent and causes direct damage to DNA. They are responsible, for example, for burns, erythemas, the increase of skin thickness and skin cancer.

UVA rays (320-400 nm) can be divided into two types of radiation: UVA2 (320-340 nm) and UVA1 (340-400 nm). They are responsible for the immediate pigmentation of the skin and delayed tanning. Although they have less energy than UVB radiation, they are also susceptible to inducing skin alteration especially in the case of sensitive skin or skin continuously exposed to solar radiation. In this sense, the UVA radiation emitted by the sun is permanent as it is filtered to a small extent. In addition, it does not produce a warning sign (erythema), therefore its effect is not noticed in the first instance. In addition to the direct damage, UVA rays are associated with indirect DNA damage since in the presence of both endogenous and environmental photosensitizers, they generate with the oxygen in the air, reactive oxygen species as a singlet or free radicals, the oxidizing action of which is very powerful. UVA rays penetrate the deeper layers of the skin and are responsible, for example, for photocarcinogenesis and photoaging.

The near visible radiation (400-450 nm) coming from the sun also entails a great danger for the skin mainly due to its low atmospheric filtration. Nevertheless, the lower energy content of this type of radiation in comparison with UV rays has caused it to not be an object of interest for experts for a long time.

As occurs on the skin, solar radiation attacks capillary fiber, altering it from the surface (cuticle) to the core (cortex). The ultraviolet and near visible radiation damage the intercell cement which ensures the cohesion of the scalps protecting the hair. As a consequence, the latter losses its resistance, strength, shine and smoothness. When the cuticle is damaged, the capillary fiber does not have any protection against external agents and suffers more intense cortex alterations: on one hand, keratin is degraded causing the hair to become more fragile and with split ends; on the other hand, melanin is also altered causing changes in the original color.

The following references provide complementary information on the adverse effects associated with the exposure to solar radiation: DeSimone, "Sunscreen and Suntan Products", Handbook of Nonprescription Drugs, 8a Ed., chapter 26, pages 533-546 (American Pharmaceutical Association, Washington, D.C.; 1986); Grove and Forbes, "A Method for Evaluating the Photoprotection Action of Sunscreen Agents Against UV-A Radiation", International Journal of Cosmetic Science 1982 , 4, 15-24; US 4,387,089; De Méo et al., "Genotoxicity of visible light (400-800 nm) and photoprotection assessment of ectoin, L-ergothioneine and mannitol and four sunscreens", Journal of Photochemistry and Photobiology B: Biology 2008, 91, 24-34; Parrish JA et al., "The Optics of Human Skin", The Journal of Investigative Dermatology 1981, 77, 13-19.

Therefore, although the immediate effects of solar radiation are related with skin tanning, both the ultraviolet radiation (UVA-UVB) and the near visible radiation represent a serious danger. It is therefore desirable that the three types of radiations are filtered, thus avoiding their harmful effects on the skin and hair.

The simplest way among the different techniques for protecting against solar radiations consists of minimizing the sun exposure time or alternatively the physical protection against their radiations by means of using, for example, clothes, hats, sunglasses or tinted glasses. However, there are a huge number of activities which required long periods of sun exposure and furthermore physical protection is not suitable in many occasions. As a result, photoprotective cosmetic compositions formed by physical filters such as pigments acting as solar rays barriers were developed. Nevertheless, there are studies which demonstrate that some physical filters act as photooxidation catalysts therefore the joint application of antioxidants to overcome said effect is being necessary. Subsequently, chemical filters were incorporated in the photoprotective compositions which in principle only absorbed UVB radiation since it was considered as the most dangerous solar radiation. In the recent times, photoprotective compositions that absorb both UVA rays and UVB rays commonly known as broad spectrum photoprotective compositions have been developed (Pathak M A, "Sunscreens: Progress and perspectives on photoprotection of human skin against UVB and UVA radiation", J Dermatol 1996, 23(11), 783-800; Gilaberte Y, Coscojuela C, Saenz de Santamaria M C, Gonzalez S, "Photoprotectores", Actas Dermosifiliogr 2003, 94(5), 271-293). Said broad spectrum compositions comprise different molecules to enable filtering a wide range of wavelengths. For example, the brand registered as Helioplex® contains avobenzone which is a UVA radiation photoprotector and oxybenzone which is a UVB radiation photoprotector, also showing certain absorbance in the UVA region. However, the broad spectrum photoprotective compositions generally have not considered the near visible radiation. In addition, its photostability is not described in detail and furthermore, there is evidence that their components are hormone disruptors. Therefore, it would be desirable to develop new photoprotective compositions which do not have these drawbacks.

Lophine (2,4,5-triphenylimidazole) can be prepared by heating hydrobenzamide at about 300˚C. It has been known for years that lophine and its derivatives are chemiluminescent, i.e., they cause light emission by means of a chemical reaction at low temperatures. Thus, the oxidation of lophine leads to the formation of an excited compound which emits light when decomposes (Philbrook, G E Photochemisty and Photobiology 1965, 4, 1175-1183).

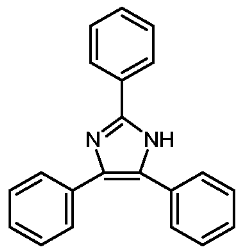

Lophine

Based on this background, it would be necessary to develop broad spectrum photoprotective compositions that are alternatives to the existing compositions for preventing or minimizing the damaging effects of solar radiation on the skin or hair which simultaneously provide protection against UVB, UVA and near visible radiations and which will furthermore have a high stability without affecting the hormone system.

## Brief Description of the Invention

The authors of the present invention have developed new cosmetic and/or pharmaceutical compositions intended for photoprotecting skin and/or hair against solar radiation, particularly against ultraviolet radiation and near visible radiation. The present invention relates to said photoprotective compositions as well as to their use in the aforementioned cosmetic and/or pharmaceutical application. More particularly, the present invention relates to photoprotective compositions that absorb in the near visible- UVA region and the UVB region comprising at least one lophine derivative of formula (I). Some of said compounds of formula (I) are soluble in polar solvents which facilitates their galenic formulation. Furthermore, their low chemical reactivity and slight decomposition due to the action of light confer stability to the photoprotective composition. In addition, the compounds of formula (I) do not have estrogenic activity, therefore the photoprotective composition can be used in subjects at risk of hormone disruption.

Therefore, in one aspect, the invention relates to a composition comprising (i) a compound A that absorbs mainly in the near visible-UVA region and (ii) a compound B that absorbs mainly in the UVB region wherein at least one of said compounds A or B is a compound of formula (I)

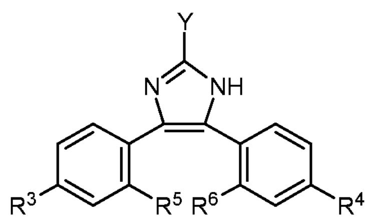

(I)

wherein
Y represents

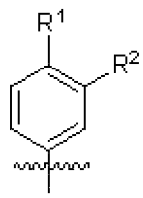

or

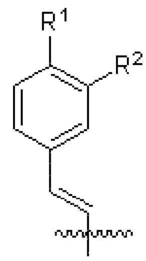

$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and $R^5$ and $R^6$ independently of each other represent H, or alternatively $R^5$ and $R^6$ together form a C-C bond between the carbon atoms to which they are bound.

In another aspect, the invention relates to a compound of formula (I")

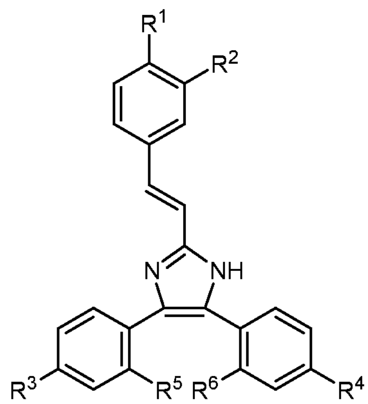

(I'')

wherein

$R^1$, $R^2$, $R^3$, $R^4$, $R^5$ and $R^6$ independently of each other represent the values defined by the formula (I).

In another aspect, the invention relates to the use of a compound of formula (I) as previously defined in the preparation of a photoprotective composition or a composition of the invention.

In another aspect, the invention relates to a cosmetic treatment method for protecting skin or hair from solar radiation or for preventing or minimizing the damaging effects produced by solar radiation on the skin or hair, which comprises applying on the skin or hair an effective amount of a composition as previously defined.

Brief Description of the Drawings

Figure 1 shows the emission spectrum of the UVB light source used during the exposure in photoprotection tests (figure provided by the manufacturer).

Figure 2 shows the response spectrum of the detector used for determining the dose applied in the photoprotection tests (figure provided by the manufacturer).

Figure 3 shows photographs depicting the appearance of the skin of the back of mice exposed to UVB on which 0.5% p-nitrolophine had been applied. The product application area is indicated with an arrow.

Figure 4 shows photographs depicting the appearance of the skin of the back of mice exposed to UVB on which 0.5% m-nitrolophine has been applied. The product application area is indicated with an arrow.

Figure 5 shows photographs depicting the appearance of the skin of the back of mice exposed to UVB on which 0.5% 4-methylbenzylidene camphor (P5000) has been applied. The product application area is indicated with an arrow.

Figure 6 shows photographs depicting the appearance of the skin of the back of mice exposed to UVB on which 0.5% butyl methoxydibenzoylmethane (P1789) has been applied. The product application area is indicated with an arrow.
Figure 7 shows a comparative spectrum of visible UV absorption of p-nitrolophine (PNLof) (black triangle), 4-methylbenzylidene camphor (Parsol 5000) (black square) and butyl methoxy-dibenzoylmethane (Parsol 1789) (black circle).
Figure 8 shows a comparative spectrum of visible UV absorption of m-nitrolophine (MNLof) (black triangle), 4-methylbenzylidene camphor (Parsol 5000) (black square) and butyl methoxydibenzoylmethane (Parsol 1789) (black circle).
Figure 9 shows a spectrum of visible UV absorption of styryl lophine.
Figure 10 depicts the average and SEM of the weight difference between the right ear and the left ear for each group of mice in the anti-inflammatory properties study: positive control (A), negative control (B) (treatment with 0.5 mg of indometacin), treatment with 0.5 mg of p-nitrolophine and (C) treatment with 1 mg of p-nitrolophine (D).

## Detailed Description of the Invention

The present invention describes photoprotective compositions useful for absorbing UVB radiation (280-320 nm) and near visible-UVA (320-450 nm). The ranges of wavelength expressed in the present invention for differentiating the different types of radiations correspond with approximate values commonly accepted in state of the art.
In the present invention the term "solar filter" refers to a compound which chemically or physically absorbs or blocks any type of ultraviolet and/or near visible radiation coming from the sun and from other natural or artificial sources. "Complementary" solar filter is any solar filter the chemical formula of which is different from formula (I).
In the present invention the term "photoprotective composition" refers to a cosmetic and/or pharmaceutical composition intended for photoprotecting the skin and/or hair against the near visible-ultraviolet radiation.
The composition of the invention comprises (i) a compound A that absorbs mainly in the near visible UVA region and (ii) a compound B that absorbs mainly in the UVB region wherein at least one of said compounds A or B is a compound of formula (I) as previously described.
In the present invention, a compound that absorbs mainly in the near visible-UVA region or UVB region refers to a compound having maximum absorption in the near visible-UVA region or UVB region, respectively. This fact does not imply that said compound also has absorption in other regions.
Although examples for preparing some representative compounds of formula (I) will be provided below, the preparation of said compounds can generally be carried out by mixing an aromatic aldehyde, a benzyl derivative and an excess of ammonium acetate and said mixture then being reacted in glacial acetic acid to reflux such as shown in the reaction scheme 1.

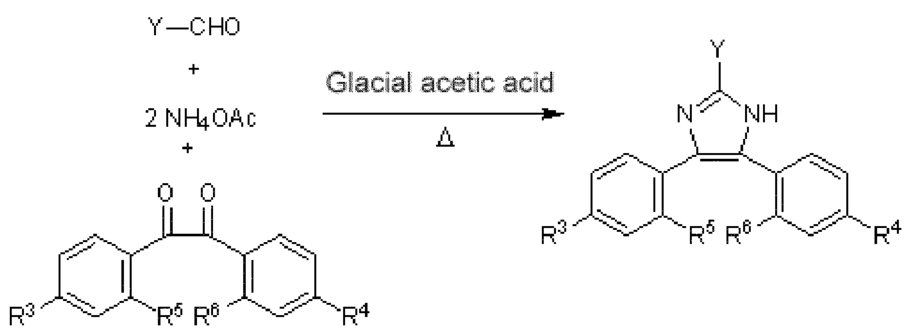


Reaction scheme 1

wherein
Y, $R^1$, $R^2$, $R^3$, $R^4$, R and $R^6$ represent the values defined for formula (I).
A process for preparing lophine mediated by microwaves as well as references to other synthesis protocols are shown in: Crouch et al., "Microwave-Mediated Synthesis of Lophine: Developing a Mechanism to Explain a Product", Journal of Chemical Education 2006, 83, 1658-1660.
In a particular embodiment, the composition of the invention comprises a single compound of formula (I). In

another particular embodiment, the composition of the invention comprises two or more compounds of formula (I). Preferably, said compound/compounds of formula (I) is/are selected from the group consisting of:

2,4,5-triphenyl-1H-imidazole;
2-(4-hydroxyphenyl)-4,5-diphenyl-1H-imidazole;
2-(4-methoxyphenyl)-4,5-diphenyl-1H-imidazole;
2-(3-nitrophenyl)-4,5-diphenyl-1H-imidazole;
2-(4-nitrophenyl)-4,5-diphenyl-1H-imidazole;
2-(4-chlorophenyl)-4,5-diphenyl-1H-imidazole;
2-(4-fluorophenyl)-4,5-diphenyl-1H-imidazole;
2-[4-(N,N-dimethylamino)phenyl]-4,5-diphenyl-1H-imidazole;
(E)-2-styryl-4,5-diphenyl-1H-imidazole;
(E)-2-(4-nitrostyryl)-4,5-diphenyl-1H-imidazole;
2,4,5-tris(4-nitrophenyl)-1H-imidazole;
2-(4-nitrophenyl)-1H-phenanthro[9,10-d]imidazole;

and mixtures thereof.

In a more particular embodiment, the composition of the invention comprises at least one compound of formula (I) selected from 2-(3-nitrophenyl)-4,5-diphenyl-1H-imidazole, 2-(4-nitrophenyl)-4,5-diphenyl-1H-imidazole, 2-[4-(N,N-dimethylamino)phenyl]-4,5-diphenyl-1H-imidazole, (E)-2-styryl-4,5-diphenyl-1H-imidazole, (E)-2-(4-nitrostyryl)-4,5-diphenyl-1H-imidazole, 2,4,5-tris(4-nitrophenyl)-1H-imidazole, 2-(4-nitrophenyl)-1H-phenanthro[9,10-d]imidazole, and mixtures thereof.

In yet a more particular embodiment, the compound of formula (I) is a compound of formula (I')

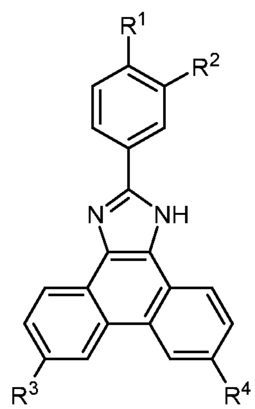

(I')

wherein
$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl.

In yet another more particular embodiment, the compound of formula (I) is a compound of formula (I")

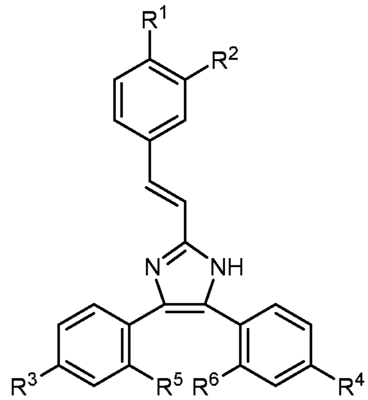

(I'')

wherein

$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and $R^5$ and $R^6$ independently of each other represent H, or alternatively $R^5$ and $R^6$ together form a C-C bond between the carbon atoms to which they are bound.

According to another particular embodiment, the composition of the invention comprises at least one compound of formula (I) that absorbs mainly near visible-UVA radiation. Preferably, said compound/compounds A of formula (I) is/are selected from 2-(4-nitrophenyl)-4,5-diphenyl-1H-imidazole, 2-[4-(N,N-dimethylamino)phenyl]-4,5-diphenyl-1H-imidazole, (E)-2-styryl-4,5-diphenyl-1H-imidazole, (E)-2-(4-nitrostyryl)-4,5-diphenyl-1H-imidazole, 2,4,5-tris(4-nitrophenyl)-1H-imidazole, 2-(4-nitrophenyl)-1H-phenanthro[9,10-d]imidazole and mixtures thereof.

According to another particular embodiment, the composition of the invention comprises at least one compound of formula (I) that absorbs mainly UVB radiation. Preferably, said compound/compounds B of formula (I) is/are selected from 2,4,5-triphenyl-1H-imidazole, 2-(4-hydroxyphenyl)-4,5-diphenyl-1H-imidazole, 2-(4-methoxyphenyl)-4,5-diphenyl-1H-imidazole, 2-(3-nitrophenyl)-4,5-diphenyl-1H-imidazole, 2-(4-chlorophenyl)-4,5-diphenyl-1H-imidazole, 2-(4-fluorophenyl)-4,5-diphenyl-1H-imidazole and mixtures thereof.

According to another more particular embodiment, the composition of the invention comprises at least one compound of formula (I) that absorbs mainly near visible-UVA radiation and at least one compound of formula (I) that absorbs mainly UVB radiation. Preferably, said compound/compounds A and B of formula (I) is/are selected respectively from:

(i) a compound A of formula (I) selected from 2-(4-nitrophenyl)-4,5-diphenyl-1H-imidazole, 2-[4-(N,N-dimethylamino) phenyl]-4,5-diphenyl-1H-imidazole, (E)-2-styryl-4,5-diphenyl-1H-imidazole, (E)-2-(4-nitrostyryl)-4,5-diphenyl-1H-imidazole, 2,4,5-tris(4-nitrophenyl)-1H-imidazole, 2-(4-nitrophenyl)-1H-phenanthro[9,10-d]imidazole and mixtures thereof; and

(ii) a compound B of general formula (I) selected from 2,4,5-triphenyl-1H-imidazole, 2-(4-hydroxyphenyl)-4,5-diphenyl-1H-imidazole, 2-(4-methoxyphenyl)-4,5-diphenyl-1H-imidazole, 2-(3-nitrophenyl)-4,5-diphenyl-1H-imidazole, 2-(4-chlorophenyl)-4,5-diphenyl-1H-imidazole, 2-(4-fluorophenyl)-4,5-diphenyl-1H-imidazole and mixtures thereof.

As previously indicated, the fact that in the present invention a compound is defined as absorbing mainly near visible-UVA radiation, or alternatively, absorbing mainly UVB radiation, does not imply that the compound does not absorb other radiations. The (E)-2-(4-nitrostyryl)-4,5-diphenyl-1H-imidazole for example has a maximum absorption in both near visible-UVA and UVB regions.

Compounds of formula (I) preferred in the composition of the invention are those wherein $R^1$, $R^2$, $R^3$ and $R^4$ independently represent H, OH, OMe, $NH_2$, NHMe, $NMe_2$, $NO_2$ fluorine or chlorine.

The composition of the invention, in addition to the at least one compound of formula (I), can comprise a complementary solar filter. Said complementary solar filter is optional when the composition of the invention comprises a compound of formula (I) that absorbs in the near visible-UVA region and other compound of formula (I) that absorbs in the UVB region. However, the presence of a complementary solar filter is necessary when the compound/compounds of formula (I) comprised in the composition only absorb in a determined region, such that said solar filter covers the other region thus offering protection in both regions.

Among the complementary solar filters suitable in the present invention are chemical and physical filters.

The chemical solar filters correspond with compounds of organic nature which absorb in a determined range

of wavelengths. It is well known that said chemical filters are classified as UVA filters and UVB filters. UVA filters mainly absorb radiation in the region between 320 and 400 nm of the spectrum. UVA filters which can act as complementary solar filters in the composition of the present invention are for example: benzophenones, camphor derivatives, dibenzoylmethane derivatives, anthranilates, bisimidazylate. UVB filters mainly absorb radiation in the region between 280 and 320 nm of the ultraviolet spectrum. UVB filters which can act as complementary solar filters in the composition of the present invention are for example: 1,3,5-triazines, cinnamates, p-aminobenzoic acid (PABA) and derivatives thereof, salicylates, camphor derivatives, benzimidazole and derivatives thereof, octocrilene and urocanic acid. There are also near visible radiation chemical solar filters and chemical solar filters capable of absorbing UVA and UVB radiation useful in the present invention, such as for example: anisotriazine, drometrizole trisiloxane, methylene bisbenzotriazolyl tetramethylbutylphenol.

The classification of chemical solar filters depending on the range of wavelengths at which they absorbs is commonly accepted in the industry. However, there is a more accurate classification depending on their chemical properties ("Sunscreens: Development, Evaluation and Regulatory Aspects" N. Shaath et al., 2a Ed, pages 269-273, Marcel Dekker, Inc. (1997)).

Physical solar filters correspond to photoprotective agents such as metal oxide pigments or nanopigments capable of physically blocking both type A and type B UV radiation as well as near visible radiation by diffusion and/or reflection. Examples of physical solar filters that can act as complementary solar filters in the composition of the present invention are optionally coated titanium, zinc, iron, zirconium, cerium oxides and mixtures thereof. These physical solar filters have been used in state of the art in different suspensions and particle sizes. The following reference offers an extensive review on physical solar filters: "Sun Protection Effect of Nonorganic Materials" S. Nakada & H. Konishi, Fragrance Journal, volume 15, pages 64-70 (1987).

Representative examples of complementary solar filters that can be formulated in the compositions of the present invention include 1,3,5-triazines, cinnamates, p-aminobenzoic acid (PABA) and derivatives thereof, salicylates, camphor derivatives, benzimidazole and derivatives thereof, octocrilene, urocanic acid, benzophenones, dibenzoylmethane derivatives, anthranilates, bisimidazylate, anisotriazine, drometrizole trisiloxane, methylene bisbenzotriazolyl tetramethylbutylphenol, titanium dioxide, zinc oxide and mixtures thereof.

Still more preferably, said complementary solar filter is selected from:

p-aminobenzoic acid (PABA),
camphor benzalkonium methosulfate,
salicylic acid ester and 3,3,5-trimethylcyclohexanol (homosalate),
benzophenone-3,
2-phenylbenzimidazole-5-sulfonic acid (potassium salt, sodium salt and triethanolamine)
terephthalylidene dicamphor sulfonic acid and salts,
methoxydibenzoylmethane butyl,
benzylidene camphor sulfonic acid and salts, octocrilene,
polyacrylamidemethyl benzylidene camphor,
octyl methoxycinnamate,
PEG-25 PABA,
isoamyl p-methoxycinnamate,
octyl triazone,
drometrizole trisiloxane,
di-octyl butamido triazone,
4-methylbenzylidene camphor,
3-benzylidene camphor,
octyl salicylate,
isopropylbenzyl salicylate,
octyl dimethyl PABA,
benzophenone-4,
benzophenone-5,
2,2'-methylene-bis-6(2h-benzotriazole-2-yl)-4-(tetramethylbutyl)-1,1,3,3-phenol,
2,2'-bis-(1,4-phenylene)-1H-benzimidazole-4,6-disulfonic acid, monosodium salt
(1,3,5)-triazine-2,4-bis{(4-2-ethyl-hexyloxy)2-hydroxy)phenyl}-6-(4-methoxyphenyl),
dimethycodiethylbenzalmalonate,
titanium dioxide , and
mixtures thereof.

Particularly, the compositions of the invention are formulated in a dosage form administered by topical route.

Said dosage forms can be both liquids and semisolids. Among the liquid dosage forms suitable to be administered by topical route are: liniment, lotion and the like. Among the semisolid dosage forms suitable to be administered by topical route are: emulsion, cream, milk, gel, gel-cream, suspension, foam, powder, ointment, pomade, paste, dressing and the like.

Preferred topical dosage forms include an emulsion of oil in water, an emulsion of water in oil and an alcohol solution.

Although individual needs mat vary, for example depending on the subject or on the type of formulation, the determination of the optimum ranges for the effective amounts of solar filters forms part of the general experience of the persons skilled in the art. Each of the solar filters included in the composition of the invention are typically in an amount between approximately 0.01% and approximately 10% and preferably between approximately 0.1% and approximately 5% by weight of the composition.

The composition of the invention typically comprises a cosmetically and/or pharmaceutically acceptable vehicle or excipient. A cosmetically and/or pharmaceutically acceptable vehicle or excipient is that for cosmetic and/or pharmaceutical use which is physiologically tolerable and does not normally cause an allergy reaction or a similar adverse reaction when it is administered into animals, more particularly into human beings. The selection of these ingredients is a routine task for the person skilled in the art depending on the type of product in which the composition is formulated. In the CTFA Cosmetic Ingredient Handbook, 7a Ed, 1997, and also in the 8a Ed, 2000, a wide variety of cosmetically and/or pharmaceutically acceptable ingredients commonly used in skin care compositions which can be incorporated in the compositions of the present invention are described. Some non-limiting examples of suitable vehicles and excipients are described below: a fat body, an organic solvent, a thickener (ionic or non ionic), a softener, an antioxidant, an opacifier, a stabilizer, an emollient, an anti-foaming agent, a moisturizing agent, a vitamin, a flavoring agent, a preservative, a surface active agent, a colorant, a sequestrant, an alkalizing agent, an acidifier agent and mixtures thereof.

According to another aspect, the invention relates to a compound of formula (I")

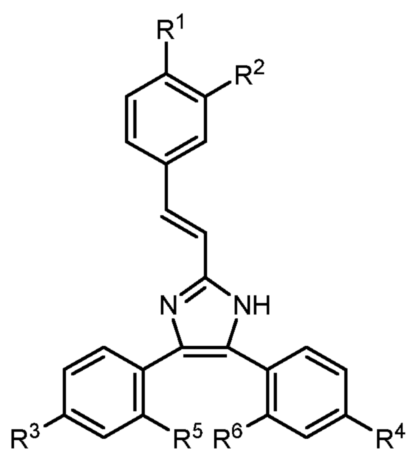

(I'')

wherein

$R^1$, $R^2$, $R^3$ and $R^4$ independently of each other represent H, OH, $OR_a$, $NH_2$, $NHR_a$, $NR_aR_b$, $N0_2$ or halogen; wherein $R_a$ and $R_b$ independently of each other represent $C_1$-$C_{10}$ alkyl; and $R^5$ and $R^6$ independently of each other represent H, or alternatively $R^5$ and $R^6$ together form a C-C bond between the carbon atoms to which they are bound.

The preferred compounds of formula (I") are those wherein $R^1$, $R^2$, $R^3$ and $R^4$ independently of each other represent H, OH, OMe, $NH_2$, NHMe, $NMe_2$, $NO_2$ fluorine or chlorine. The yet more preferred compounds of formula (I'') are those wherein $R^1$, $R^2$, $R^3$ and $R^4$ independently of each other represent H, $NMe_2$ or $NO_2$. Particular examples of the preferred compounds of formula (I") include (E)-2-styryl-4,5-diphenyl-1H-imidazole and (E)-2-(4-nitrostyryl)-4,5-diphenyl-1H-imidazole.

The compounds of formula (I") have a double bond conjugated between the central ring of imidazole and a phenyl. This increase of electron delocalization favors the absorption at greater λ, i.e., more towards the visible wavelengths. In this sense, the compounds of formula (I") are specially useful for achieving a bathochromic effect, thus providing photoprotection in greater wavelengths in comparison to the compounds which do not have said conjugated double bond.

According to another aspect, the invention relates to the use of a compound of formula (I) as previously defined in the preparation of a photoprotective composition.

In a particular embodiment, the compound of formula (I) used in the preparation of the photoprotective composition is selected from the group consisting of:

2,4,5-triphenyl-1H-imidazole;
2-(4-hydroxyphenyl)-4,5-diphenyl-1H-imidazole;
2-(4-methoxyphenyl)-4,5-diphenyl-1H-imidazole;
2-(3-nitrophenyl)-4,5-diphenyl-1H-imidazole;
2-(4-nitrophenyl)-4,5-diphenyl-1H-imidazole;
2-(4-chlorophenyl)-4,5-diphenyl-1H-imidazole;
2-(4-fluorophenyl)-4,5-diphenyl-1H-imidazole;
2-[4-(N,N-dimethylamino)phenyl]-4,5-diphenyl-1H-imidazole;
(E)-2-styryl-4,5-diphenyl-1H-imidazole;
(E)-2-(4-nitrostyryl)-4,5-diphenyl-1H-imidazole;
2,4,5-tris(4-nitrophenyl)-1H-imidazole; and
2-(4-nitrophenyl)-1H-phenanthro[9,10-d]imidazole.

In another aspect, the invention relates to a compound of formula (I) for use in photoprotection.

Likewise, the compound of formula (I) can be used in the preparation of a photoprotective composition in combination with other compound or compounds of formula (I), as well as with one or more complementary solar filters.

Both from the pharmaceutical and cosmetic point of view, the photoprotective composition comprising at least one compound of formula (I) is used to protect skin or hair from solar radiation. As discussed in the background, the damaging solar radiation reaching the Earth is composed of UVB rays, UVA rays and near visible radiation. As a result, the photoprotective composition of the invention is useful for protecting skin or hair from the type A and type B and near visible ultraviolet radiation of the sun. Nevertheless, the use of the photoprotective composition of the invention is not solely limited to solar radiation but also include other types of radiations both of natural and artificial origin comprising UVB rays, UVA rays and/or near visible radiation. Among the artificial radiation sources are, for example, radiations received in hospitals, industries, cosmetic treatments (UVA rays tanning beds), etc.

The invention also relates to a cosmetic treatment method for protecting skin or hair from solar radiation or other radiation of a natural or artificial origin or for preventing or minimizing the damaging effects produced by solar radiation or other radiation of a natural or artificial origin on the skin or hair, which comprises applying on the skin or hair an effective amount of a composition as previously defined.

There are various damaging effects produced by solar radiation or other radiation of a natural or artificial origin on the skin or hair. The photoprotective composition of the invention can be used in the field of cosmetics or with a pharmaceutical purpose for preventing or minimizing such effects like for example erythema, severe sunburns, sun-induced premature aging of the skin, regulation of biochemical deterioration caused by free radicals, photocarcinogenesis, melanoma, non-melanoma skin cancer, etc.

Therefore, the photoprotective composition is applicable both on healthy subjects in order to prevent the damaging effects produced by solar radiation or other radiation of a natural or artificial origin, as well as on subjects affected by any of those effects in order to minimize them.

The compounds of formula (I) do not have estrogenic activity and therefore can be used by subjects at risk of hormone disruption. In a particular embodiment the photoprotective composition is used for preventing or minimizing the damaging effects produced by solar radiation or other radiation of a natural or artificial origin on the skin or hair in subjects at risk of hormone disruption such as children and pregnant women.

The following examples illustrate the invention, they allow the person skilled in the art to carry out the present invention and they should not be considered in a limiting manner thereof.

## EXAMPLES

In the following examples, "ppa" means "pure product for analysis".

### EXAMPLE 1

### Synthesis of compounds of formula (I)

#### 1.1 General process for synthesis

Lophine and its monosubstituted derivatives p-hydroxy ($R^1$= OH), p-methoxy ($R^1$= OMe), m-nitro ($R^2$= $NO_2$), p-nitro ($R^1$= $NO_2$), p-chlorine ($R^1$= Cl) and p-fluorine ($R^1$= F) were prepared following the general process described in

the reaction scheme 1. More specifically, a mixture of corresponding aldehyde (1 mmol), benzyl (210 mg, 1 mmol) and an excess of ammonium acetate (350 mg, 4.5 mmoles) were heated to reflux in glacial acetic acid for 4 hours. The mixture was cooled and 30 mL of water were added using an equalizer. The precipitate was filtered, washed with water and dried in a dryer. The product was crystallized in ethanol.

1.2 Synthesis of 2-[4-(N,N-dimethylamino)phenyl]-4,5-diphenyl-1H-imidazole

30 mL of glacial acetic acid, 0.64 g (3.06 mmol) of benzyl and 1.08 g (13.90 mmol) of ammonium acetate were placed in a two-necked flask with coolant. The bath was heated and once reflux was reached 0.44 g (2.99 mmol) of p-dimethylamino benzaldehyde were added, maintaining the reflux for 4 hours. The reaction was left to cool and was then extracted using ethyl acetate as a solvent. The organic phase was dried by rotary evaporation. 335 mg of the crude reaction product were taken, dissolved in chloroform for seeding and were purified by flash silica column using petroleum ether:ethyl acetate (1:1) as eluent and 95 mg of pure product (28.4%) were obtained.

1.3 Synthesis of (E)-2-styryl-4,5-diphenyl-1H-imidazole

30 mL of glacial acetic acid, 0.66 g (3.1 mmol) of benzyl and 1.08 g (13.90 mmol) of ammonium acetate were placed in a two-necked flask with coolant. The bath was heated and once reflux was reached 0.42 g (3.2 mmol) of cinnamaldehyde were added, maintaining the reflux for 3 hours. The reaction was left to cool and was then emptied into a water/ice bath. The precipitate was filtered under vacuum and put back into ethyl acetate. The organic phase was dried by rotary evaporation. 868 mg of the crude reaction product were taken and purified by flash silica column using petroleum ether:ethyl acetate (4:1) as eluent and 422 mg of pure product (42.2%) were obtained.

1.4 Synthesis of (E)-2-(4-nitrostyryl)-4,5-diphenyl-1H-imidazole

30 mL of glacial acetic acid, 0.64 g (3.06 mmol) of benzyl and 1.08 g (13.90 mmol) of ammonium acetate were placed in a two-necked flask with coolant. The bath was heated and once reflux was reached 0.44 g (2.5 mmol) of p-nitrocinnamaldehyde were added, maintaining the reflux for 1 and a half hours. The reaction was left to cool and was emptied into a water/ice bath. The precipitate was filtered under vacuum, washed with hot water and dried in a dryer. 503 mg of the crude reaction product were taken and purified by flash silica column using petroleum ether:ethyl acetate (1:1) as eluent and 265 mg of pure product (52.7%) were obtained.

1.5 Synthesis of 2,4,5-tris(4-nitrophenyl)-1H-imidazole

202 mg of p-nitrolophine obtained according to the above general synthesis process, 41 mg of urea, 0.62 mL of 98% $H_2SO_4$ were placed in a two-necked flask with coolant and bromine ball, in an oil bath and 0.50 mL of 70% $HNO_3$ were added drop-wise at room temperature. The bath was slowly heated until reaching a temperature of 63°C which was maintained for 45 minutes. The reaction was left to cool and was then emptied into water-ice. Subsequently, the precipitate was filtered under vacuum, washed with hot water and dried in a dryer to obtain 246 mg of the crude reaction product (96.5%). A sample of 52 mg of the crude product was purified by means of flash silica column using petroleum ether:ethyl acetate (3:1) as eluent and 16 mg of pure product (30%) were obtained.

1.6 Synthesis of 2-(4-nitrophenyl)-1H-phenanthro[9,10-d]imidazole

100 mL of glacial acetic acid with 1.53 g (0.001 mol) of p-nitro benzaldehyde were heated to reflux. Once reflux was reached, 2.08 g (0.001 mol) of 9,10-phenanthroquinone and 1.55 g (0.002 mol) of ammonium acetate (in excess) were added. It was left to reflux for 1 hour. It was then left to cool and was filtered. 150 mL of water and $NH_4OH$ were added to the mother liquors until neutralization. The precipitate formed was filtered and pooled with the previous one, washing with water. 360 mg were taken from the crude reaction product for purifying by flash silica column to obtain 69 mg of product (17.52%).

Table 1 shows the appearance of the solids obtained, the yield thereof, as well as the wavelengths of the maximum absorption (nm) and their corresponding absorbance (absorbance units) [UV spectrum obtained in a Cecil 2021 UV-Visible Spectrophotometer equipment].

Table 1

| Product | Appearance | Yield | UV spectrum | |
|---|---|---|---|---|
| | | | λ (nm) | A (a.u) |
| 2,4,5-triphenyl-1H-imidazole (Lophine) | white | 98% | 209.0<br>296.0 | 0.817<br>1.093 |
| 2-(4-hydroxyphenyl)-4,5-diphenyl-1H-imidazole (4OH- Lophine) | yellowish | 76% | 225.0<br>300.5 | 1.015<br>1.364 |
| 2-(4-methoxyphenyl)-4,5-diphenyl-1H-imidazole (4MeO-Lophine) | yellowish | 81% | 252.5<br>297.5 | 0.240<br>1.019 |
| 2-(3-nitrophenyl)-4,5-diphenyl-1H-imidazole ($3NO_2$-Lophine) | yellowish | 81% | 309.0 | 1.194 |
| 2-(4-nitrophenyl)-4,5-diphenyl-1H-imidazole ($4NO_2$-Lophine) | yellow | 90% | 221.0<br>255.0<br>379.0 | 0.715<br>0.669<br>0.774 |
| 2-(4-chlorophenyl)-4,5-diphenyl-1H-imidazole (4Cl-Lophine) | white | 98% | 231.5<br>308.0 | 1.066<br>1.371 |
| 2-(4-fluorophenyl)-4,5-diphenyl-1H-imidazole (4F-Lophine) | yellow | 76% | 224.0<br>297.5 | 1.117<br>1.536 |
| 2-[4-(N,N-dimethylamino)phenyl]-4,5-diphenyl-1H-imidazole | orangish | 28% | 209.5<br>322 | 2.032<br>2.692 |
| (E)-2-styryl-4,5-diphenyl-1H-imidazole (Styryl Lophine) | yellowish | 42% | 235.2<br>344.4 | 0.672<br>1.643 |
| (E)-2-(4-nitrostyryl)-4,5-diphenyl-1H-imidazole | yellowish | 53% | 202.0<br>282.5<br>398.5 | 0.326<br>0.122<br>0.169 |
| 2,4,5-tris(4-nitrophenyl)-1H-imidazole | yellowish/ golden | 30% | 236.0<br>370.0 | 1.410<br>1.923 |
| 2-(4-nitrophenyl)-1H-phenanthro[9,10-d]imidazole | orangish | 18% | 202.0<br>256.0<br>388.5 | 1.329<br>2.412<br>0.922 |

All the above monosubstituted lophine derivatives, with the exception of m-nitrolophine, are very soluble or soluble in solvents such as ethyl acetate or acetone.

EXAMPLE 2

Photostability evaluation for compounds of formula (I)

All the irradiations preformed in the photostability studies were carried out in an ACE 7840 photoreactor using a 450W power ACE 7825 lamp.

2.1 Photostability evaluation for 2-(4-nitrophenyl)-4,5-diphenyl-1H-imidazole ($4NO_2$-Lophine or p-nitrolophine)

As a preliminary step, in the case of p-nitrolophine the specific molar absorptivity of the molecule (ε) ($A^1_1$) was determined. To that end, 50.4 mg of p-nitrolophine were weight, they were dissolved in 100.0 mL of MeOH (ppa); 4 dilutions were then performed by taking 1.0, 2.0, 5.0 and 10.0 mL from this mother liquor and topping up each of them to a volume of 100.0 ml with MeOH (ppa). Table 2 shows the absorbance values for each of the samples.

Table 2

| Concentration (mol) | Abs. at 379 nm (A.U.) |
|---|---|
| 0.0000147 | 0.2572 |
| 0.00002953 | 0.5214 |
| 0.00007382 | 1.301 |
| 0.00014764 | 2.4819 |

The slope of the straight line obtained from these points (Y= 16.717x + 0.03; R2 = 0.9993) therefore coincides with the value of specific molar absorptivity of p-nitrolophine at 379 nm

$$\varepsilon_{(379\ nm)} = 16.717\ A.U.L/mol$$

The solvent used in the photostability test was methanol [MeOH] (ppa) (250 mL), using a concentration of 1 g/mL of p-nitrolophine. The solution was irradiated for 2 hours, samples being taken at 10 minutes, 20 minutes, 1 hour and 2 hours, and being dried in a rotating evaporator. Each sample was analyzed by thin layer chromatography (TLC in Silica gel) using petroleum ether:ethyl acetate (4:1) as eluent. The chromatography plates were viewed under a 254 nm lamp and a 366 nm lamp, as well as by means of simultaneous viewing under both lamps (254 and 366 nm).

Once the experiment ended (2 hours) there was a high concentration of the starting product which indicates that the photodecomposition is slow. Specifically, three photoproducts (three blots) could be determined, which were isolated by preparative TLC. An approximate estimation of the total sum of said photoproducts indicates that the p-nitrolophine had suffered a degradation lower than 5%. Furthermore, the number of blots which appeared on the plate, i.e. 3, was constant, demonstrating that the isolated photoproducts did not undergo secondary photodecomposition.

2.2 Photostability evaluation for 2-(3-nitrophenyl)-4,5-diphenyl-1H-imidazole ($3NO_2$-Lophi or m-nitrolophine)

A saturated solution of m-nitrolophine in MeOH was prepared to perform the study since the solubility in this solvent is low (approximately 0.2 mg/mL). Briefly, an amount of m-nitrolophine sufficient to ensure the saturation in 200 mL of MeOH throughout the night was left stirring. The solution was filtered and irradiated for 2 hours, samples of 10 mL being taken at 10 minutes, 20 minutes, 1 hour and 2 hours, and being dried in rotating evaporator. Each sample was analyzed by thin layer chromatography (TLC in silica gel) using petroleum ether:ethyl acetate (2:1) as eluent and it was viewed under a UV lamp at 254 nm.

After 2 hours only a blot of interest appeared which was isolated. To that end, the product was taken after 2 hours of irradiation, dried by rotary evaporation and purified by means of column chromatography (flash silica) using the mixture used to perform TLC as eluent, resulting in 5.7 mg of photodecomposition product. Furthermore, the absence of new blots indicates that there is no secondary photodecomposition.

EXAMPLE 3

Evaluation of estrogenic hormonal activity

3.1 Evaluation of estrogenic hormonal activity of 2-(4-nitrophenyl)-4,5-diphenyl-1H-imidazole (p-nitrolophine)

In order to evaluate the possible estrogenicity of p-nitrolophine, its capacity of binding to estrogenic receptors and activating the expression of specific genes for hormonal activity was investigated using different in vitro estrogenicity bioassays with known sensitivity and specificity.

Briefly, the transactivation bioassay (Reporter gene assay) used are based on the use of breast and uterine cancer cell lines transfected with different native or modified hormone receptors. The resulting cell lines further contain a reporter gene regulated by estrogens through the estrogen response element, the globin promoter and the luciferase gene. The assembly obtained specifically responds to the stimulus from natural estrogen (estradiol) or from any estrogen mimicking compounds inducing the expression of the reporter gene which clearly gives rise to a colorimetric reaction.

Materials and methods

Chemical compounds

Stock solutions (10 nM) in ethanol (EtOH) for both p-nitrolophine and estradiol were prepared, they were kept at - 20˚C until use thereof. The successive dilutions were performed in the culture medium.

The cell culture media and fetal bovine serum (FBS) were obtained from Life Technologies Inc. The antibiotic geneticin and luciferin were bought from Promega. The 17β-estradiol ($E_2$) and the antibiotic puromycin were bought from Sigma- Aldrich Inc.

All the plastic material for the culture was obtained from Falcon (Merck Eurolab) with the exception of the 96 well plates which were obtained from Greiner Labortechnik.

Cell lines and culture conditions

MELN cells were obtained by means of transfecting the MCF-7 breast cancer cell line [which endogenously express the estrogen receptor alpha (ERα)] with an estrogen sensitive response element (ERE-βGlob-Luc-SV-Neo). The selection of clones resistant to geneticin was performed using a concentration of 1 mg/mL. The maintenance medium for this cell line is DMEM F12 (Dulbecco's modified Eagle medium) with phenol red supplemented with 10% FBS, 1% of penicillin/streptomycin and 1 mg/mL of geneticin. Due to the estrogenic activity of the phenol red and FBS, the experiments are preformed in medium known as experimental medium, i.e., a culture medium without phenol red and with FBS lacking of estrogens. The experimental medium for this line is DMEM without phenol red supplemented with 3% of FBS treated with carbon-dextran. The basal activity of these cells obtained with 10 nM of $E_2$ is about 15% of the maximum activity.

HELN-ERα and HELN-ERβ cell lines were obtained by means of transfecting the HeLa uterine cancer cell line. In a first phase, said cells were transfected with an estrogen sensitive response element and then with the estrogen receptor alpha or beta (ERα or ERβ). The selection of clones resistant to geneticin and puromycin was performed using a concentration of 1 mg/mL and 0.5 mg/mL, respectively. The maintenance medium as well as the experimental medium of these 2 cell lines is DMEM F12 without phenol red supplemented with 6% of FBS (lacking of estrogens), 1% of penicillin / streptomycin, 1 mg/mL of geneticin and 0.5 μg/mL of puromycin. The basal activity of these cells obtained with 10 nM of $E_2$ is about 10% of the maximum activity.

Estrogenicity bioassay: luciferase activity

The cells were seeded at a density of $5x10^4$ cells per well in white opaque 96 well plates in 150 μL of experimental medium. After 8 hours, the compounds to be assayed (p-nitrolophine and estradiol) were prepared (concentrated 4x) in the same medium and were added to each well (50 μl). The cells were incubated for 16 hours at 37˚C. At the end of the incubation, the medium was removed and replaced with fresh medium containing 0.3 nM luciferin [at that concentration, luciferin diffuses into cell producing a luminescent signal which is stable after 5 minutes and for at least 2 hours]. The plates were then introduced in the luminometer to measure the luminescence for 2 seconds. A YicroBeta Trilux luminometer (EGG Wallac) apparatus was used to detect luciferase activity.

Analysis of the results

The assays were preformed in quadruple for each concentration (0.01-10 μM) in at least three independent experiments. The results were expressed as the percentage of maximum luciferase activity (100%) which corresponded to that obtained in the presence of 10 nM $E_2$. The values of luciferase activity obtained were analyzed using the one-way ANOVA statistic test. The differences were considered significant when P was less than 0.5 ($P < 0.05$).

Results and Discussion

First, in order to investigate the estrogenicity of p-nitrolophine the MELN cell line commonly used to identify the hormonal activity of compounds interacting specifically with the estrogen receptor alpha, has been used. As shown in Table 3, p-nitrolophine was not capable of inducing the expression of luciferase beyond the value obtained with the negative control (hormone free culture medium) even at the highest assayed concentration (10 μM). This assay proofs that p-nitrolophine is not an estrogenic compound.

Table 3

| Transcriptional activity of ERα in response to p-nitrolophine ($pNO_2$ Lophine) | |
|---|---|
| Compound | Luciferase activity (%) |
| Positive control $E_2$ ($1x10^{-8}$ M) | 100 |
| Negative control (Culture medium) | 15 ± 0.8 |
| pNO Lophine ($1x10^{-8}$ M) | 15.2 ± 0.6 |
| $pNO_2$ Lophine ($3x10^{-8}$ M) | 15.3 ± 0.5 |
| $pNO_2$ Lophine ($1x10^{-7}$ M) | 15.6 ± 0.3 |
| $pNO_2$ Lophine ($3x10^{-7}$ M) | 15.5 ± 0.9 |
| $pNO_2$ Lophine ($1x10^{-6}$ M) | 16 ± 0.4 |
| $pNO_2$ Lophine ($3x10^{-6}$ M) | 15.2 ± 0.4 |
| $pNO_2$ Lophine ($1x10^{-5}$ M) | 15.6 ± 0.9 |

The MELN cells were treated with said compound at the indicated concentrations. The results are expressed as the percentage of maximum luciferase activity (100%) which was that obtained in the presence of 10 nM $E_2$

In order to confirm the results obtained and to categorize the estrogenic profile of p-nitrolophine, the product was then assayed in another two bioassays based on the use of HELN-ERα and -ERβ cell lines by means of which whether the estrogenicity depends on estrogen receptors alpha or beta, respectively, can be differentiated. The results obtained in the bioassay based on which HELN-ERα line is used again indicate that the compound is not estrogenic (Table 4), thus confirming the previous results.

Table 4

| Transcriptional activity of ERα in response to p-nitrolophine ($pNO_2$ Lophine) | |
|---|---|
| Compound | Luciferase activity (%) |
| Positive control $E_2$ ($1x10^{-8}$ M) | 100 |
| Negative control (Culture medium) | 10.7 ± 1.3 |
| $pNO_2$ Lophine ($1x10^{-8}$ M) | 11.1 ± 0.9 |
| $pNO_2$ Lophine ($3x10^{-8}$ M) | 11.7 ± 0.9 |
| $pNO_2$ Lophine ($1x10^{-7}$ M) | 10.7 ± 1.1 |
| $pNO_2$ Lophine ($3x10^{-7}$ M) | 11.3 ± 1.5 |
| $pNO_2$ Lophine ($1x10^{-6}$ M) | 11.1 ± 1.1 |
| $pNO_2$ Lophine ($3x10^{-6}$ M) | 11.2 ± 1.4 |
| $pNO_2$ Lophine ($1x10^{-5}$ M) | 11.6 ± 1.5 |

The HELN-ERα cells were treated with said compound at the indicated concentrations. The results are expressed as the percentage of maximum luciferase activity (100%) which was that obtained in the presence of 10 nM $E_2$.

In addition, neither does p-nitrolophine induce an increase in the luciferase activity when it is assayed in the bioassay based on the use of HELN-ERβ line (Table 5). Therefore, this assay also points out that p-nitrolophine is not an estrogenic compound.

Table 5

| Transcriptional activity of ERα in response to p-nitrolophine ($pNO_2$ Lophine) | |
|---|---|
| Compound | Luciferase activity (%) |
| Positive control $E_2$ ($1x10^{-8}$ M) | 100 |
| Negative control (Culture medium) | 9.5 ± 1.1 |
| $pNO_2$ Lophine ($1x10^{-8}$ M) | 9.6 ± 1.4 |
| $pNO_2$ Lophine ($3x10^{-8}$ M) | 10.0 ± 0.8 |

(continued)

| Transcriptional activity of ERα in response to p-nitrolophine ($pNO_2$ Lophine) | |
|---|---|
| Compound | Luciferase activity (%) |
| $pNO_2$ Lophine ($1x10^{-7}$ M) | 10.2 ± 1.3 |
| $pNO_2$ Lophine ($3x10^{-7}$ M) | 10.3 ± 1.1 |
| $pNO_2$ Lophine ($1x10^{-6}$ M) | 10.1 ± 1.3 |
| $pNO_2$ Lophine ($3x10^{-6}$ M) | 10.2 ± 1.9 |
| $pNO_2$ Lophine ($1x10^{-5}$ M) | 10.6 ± 1.2 |
| The HELN-ERβ cells were treated with said compound at the indicated concentrations. The results are expressed as the percentage of maximum luciferase activity (100%) which was that obtained in the presence of 10 nM $E_2$ | |

Given the sensitivity and specificity of the methodology used, the absence of estrogenic activity in estrogenicity tests in culture allows concluding that it is very improbable that the p-nitrolophine will have estrogenic behavior in animal models.

3.2 Evaluation of the estrogenic hormonal activity of 2-(3-nitrophenyl)-4,5-diphenyl-1H-imidazole ($3NO_2$-Lophine or m-nitrolophine)

In order to evaluate the possible estrogenic hormonal activity of m-nitrolophine, its capacity of binding to estrogen receptor and activating cell proliferation after 144 hours of subculture in the absence (negative control) or presence (positive control) of 17β-estradiol by means of an in vitro estrogenicity test (E-Screen) using the breast cancer cell line known as MCF-7 [Experiments of cell proliferation: E-Screen Test], was investigated.

The E-Screen proliferation test is based on the use of MCF-7 breast cancer cell line. The MCF-7 cells [which endogenously expressed the estrogen receptor alpha (ERα)], respond to the treatment with estradiol increasing its proliferative rate, synthesizing new proteins and transcribing certain specific genes. These cells are considered as estrogen-dependents, whereby they do not proliferate when they are exposed to a culture medium supplemented with serum from which estrogens have been removed, only natural or synthetic estrogens annul this inhibition and induce maximum proliferation. By taking advantage of these characteristics Soto et al. ["An in culture bioassay to assess the estrogenicity of xenobiotics"; in Chemically Induced Alterations in Sexual Development: The Wildlife/Human Connection; Colborn T, Clement CR, eds; Princeton, NJ: Princeton Scientific Publishing, 295-309; 1992] developed a biological assay under the name of E-Screen (US 4,859,585). The test compares the number of cells or the cell proliferation obtained after 6 days of culture. The cells grow in culture medium supplemented with human serum lacking of estrogens, in the presence and/or absence of estradiol as well as of the chemical compounds suspected of having estrogenic activity.

Materials and methods

Chemical compounds

A 30 mg/ml stock solution of m-nitrolophine in ethanol was prepared and assayed in the range of 300-0.003 μg/ml. In addition, stock solutions of 17β-estradiol (10 mM) in ethanol were also prepared and kept at -20˚C until use thereof. The successive dilutions of both the problem compound and estradiol were preformed in the culture medium.

The cell culture media and the fetal bovine serum (FBS) were obtained from Life Technologies Inc. Estradiol-17β, L-glutamine, Hepes and sodium bicarbonate were bought from Sigma-Aldrich Inc. All the plastic material for the culture was obtained from Falcon (Merck Eurolab) with the exception of the 24 well plates which were obtained from Greiner Labortechnik.

Cell lines and culture conditions

The MCF-7 human cancer cell line is used. This line was established by Soule et al. ["A human cell line from a pleural effusion derived from a breast carcinoma". J. Natl. Cancer Inst. 51:1400-1413; 1973] from a human breast carcinoma. Its huge diffusion as experimental model for breast cancer can be attributed to the fact that it is the first cell line documented as positive estrogenic receptor which responds to metabolic and structural changes due to the action of the estrogens [Soto et al. "The E-Screen as a tool to identify estrogens: an update on oestrogenic environment

pollutants". Environ. Health Perspect. 103:113-122, 1995]. The MCF-7 cell line further has specific receptors for other hormonal agents among which are androgens, progesterones, glucocorticoids, vitamin D3, thyroid hormones, prolactin, insulin, calcitonin and cell growth stimulating factors.

In the present study, the stock from MCF-7 BUS cells yielded by Dr. C. Sonnenschein (Tufts University, Boston, USA.) and cloned as C7MCF-7 from the passage 173 of the original MCF-7 yielded by Dr. McGrath of Michigan Cancer Foundation, was used.

As a nutrient medium for the assayed cell line, the following synthetic media were used:

a) *Maintenance medium*: The maintenance of the in culture MCF-7 cells was carried out in a minimum essential medium DMEM (Dulbecco's modified Eagle medium) supplemented with 10% of FBS plus phenol red (PR), L-glutamine and sodium bicarbonate; and

b) *Experimental medium*: Due to the estrogenic activity of phenol red and FBS, the experiments are preformed in medium known as experimental medium, i.e., culture medium without phenol red being indicated in this case as DMEM without PR in the text. In this case, the pH regulation of the medium was carried out using sodium bicarbonate and Hepes as buffer at final concentrations of 4.2 μl and 20 mM, respectively. The experimental medium was supplemented with 10% of human serum lacking of estrogens (10% CDHuS).

Estrogenicity bioassay

The E-Screen test was performed following the methodology previously described by Soto et al. ["An in culture bioassay to assess the estrogenicity of xenobiotics. In: Chemically Induced Alterations in Sexual Development: The Wildlife/Human Connection; Colborn T, Clement CR, eds; Princeton, NJ: Princeton Scientific Publishing, 295-309; 1992] modified by Villalobos et al. ["The E-SCREEN assay: comparison among different MCF7 cell stocks, Environ. Health Perspect. 103:844-850; 1995]. The in confluence MCF-7 cells were trypsinized and the aliquots of said suspensions were seeded in 24 well plates at the initial concentrations of 20,000-40,000 cells per well in maintenance medium DMEM with PR, supplemented with the 10% of FBS. When the cells were adhered to the plastic support of the plate (generally 24-48 hours) the maintenance medium was removed and the experimental medium DMEM without PR supplemented with the 10% of CDHuS was added. 17β-estradiol and the compounds to be assayed were added to the culture medium at the required concentrations. The assay was ended after 144 hours of subculture (exponential phase) after drawing out the medium and fixing the cells for the application of sulforhodamine-B technique. Finally, the cell growth for each experimental group is related to the growth obtained for the negative control (absence of hormone) and with respect to estradiol (positive control).

Analysis of the results: Parameters for biological activity

Once the E-Screen test is performed, the maximum cell proliferation rate induced by the compound also known as proliferative effect (PE) was established by calculating the ratio existing between the maximum proliferation rate obtained for the compound and the proliferation rate of the negative control. The proliferation data corresponding with the successive dilutions of the compound were transformed after the application of the correction factor for the dilution of each experimental point, the arithmetic mean of the data obtained being calculated. Another parameter is the relative proliferative efficiency (RPE). To that end, the maximum cell proliferation rate obtained was divided by the maximum cell proliferation rate of the 17β-estradiol (which is about 6.2 ± 0.3 over control). The resulting value was therefore expressed in percentage, estimating 100% for 17β-estradiol. Finally, the relative proliferation potential (RPP) was also estimated by calculating the ratio existing between the concentration at which the assayed product presented the maximum proliferative capacity and that at which the 17β-estradiol presented its maximum proliferative effect. The resulting value was therefore expressed in percentage, estimating 100% for 17β-estradiol. When the relative proliferative effect of a compound or mixture thereof is about 100 it will considered as a full agonist, if the value is about 1 it will considered as a compound lacking of estrogenic activity, for intermediate values it pertains to partial agonists.

Results and Discussion

In this assay, the estrogenicity E-Screen test commonly used to identify the hormonal activity of compounds interacting specifically with the estrogen receptor alpha (ERα), has been used. The assays were performed in triplicate for each concentration (range: 300-0.003 μg/ml) in at least three independent experiments.

The results expressed as proliferative effect obtained after performing the bioassay (Table 6) clearly indicate that m-nitrolophine does not induce a significant cell proliferation with respect to the negative control (hormone free culture medium), having a proliferative effect lower than 1.5 in all the tested concentrations range. Generally, a compound is considered to have estrogenic activity when its proliferative effect is 2 or more than 2, therefore it can be considered

that m-nitrolophine is not an estrogenic compound for the receptor alpha.

Table 6

| Relative cell growth obtained in the E-Screen bioassay for m-nitrolophine ($mNO_2$ Lophine) | | |
|---|---|---|
| Proliferative effect (PE) | | |
| | Mean | Standard Deviation |
| Positive control $E_2$ ($1x10^{-1}$ M) | 6, 62* | 0.41 |
| Negative control (Culture medium) | 1 | 0.11 |
| $mNO_2$ Lophine ($3x10^{-1}$ mg/ml) | 1.45 | 0.10 |
| $mNO_2$ Lophine ($3x10^{-2}$ mg/ml) | 1.30 | 0.07 |
| $mNO_2$ Lophine ($3x10^{-3}$ mg/ml) | 1.19 | 0.13 |
| $mNO_2$ Lophine ($3x10^{-4}$ mg/ml) | 1.10 | 0.12 |
| $mNO_2$ Lophine ($3x10^{-5}$ mg/ml) | 1.08 | 0.09 |
| $mNO_2$ Lophine ($3x10^{-6}$ mg/ml) | 1.06 | 0.13 |

*: Significant difference with respect to the control estimating a growth equal to 1; $p< 0.05$ The MCF-7 cells were treated with said compound at the indicated concentrations. The results are expressed as the maximum cell proliferation rate induced by the compound (proliferative effect), calculated as the ratio existing between the maximum proliferation rate obtained for the compound and the proliferation rate achieved by the negative control.

EXAMPLE 4

Evaluation of photoprotection

The effect of p-nitrolophine and m-nitrolophine on erythema due to exposing the mouse skin to an irradiation source at maximum in the UVB region has been studied in these assays. The results obtained have been compared with the effect of 4-methylbenzylidene camphor (P5000) a substance considered as a UVB solar filter and with the effect of butyl methoxydibenzoylmethane (P1789) an UVA type filter, both products being commonly used in antisolar cosmetics.

Materials and Methods

35 male mice weighing more than 20 grams have been used. After receiving them, the animals were acclimated for 7 days at the place where the assays were preformed, they were being placed in a cage with controlled temperature (22˚C), with relative humidity between 50% and 75%, replacing fresh filtered air approximately 10 times per hour and with 12 h light/dark cycles (7.00 - 19.00 light and from 19.00 to 7.00 dark). For this period and during the experimental period, the animals were fed *ad libitum* with standard diet for rodents and running water as drink.

The animals were randomly distributed into 4 experimental groups according to Table 7. The products to be tested were directly used, applying them in the form of a gel on an area of the skin such that each animal form its own control.

Table 7

| Group | Treatment | Concentration | Exp to UVB | Dose | No. of animals | Admin route |
|---|---|---|---|---|---|---|
| 1 | p-nitrolophine gel* | 0.5% | Yes | 100 ml. in 2x2 cm | 10 | Topical |
| 2 | m-nitrolophine gel** | 0.5% | Yes | 100 ml. in 2x2 cm | 10 | Topical |
| 3 | P5000 gel* | 0.5% | Yes | 100 ml. in 2x2 cm | 10 | Topical |
| 4 | P1789 gel* | 0.5% | Yes | 100 ml. in 2x2 cm | 5 | Topical |

* 10 mg of substance + 50 mg of carbopol 941 dissolved in 2 ml of ethanol and stirred until gelled.
** 10 mg of m-nitrolophine in 2 ml of a mixture of solvents (300 ml of octyl-dodecanol + 300 ml of hexylene glycol + 400 ml of methanol)

The test was performed following the method described by Winder et al. ["A study of Pharmacological influences

on ultraviolet erythema in guinea pigs". Arch. Int Pharmacodyn, 116:261-292; 1958] and by other authors [Wendy et al. "The local antinociceptive and topical antiinflamatory effects of propyl gallate in rodents". Br. J. Pharmacol, 58:573 -581; 1976; Katiyar et al. "Protective Effects of Silymarin Against Photocarcinogenesis in a Mouse Skin Model". J Natl Cancer Inst 89:556-65; 1997] with slight modifications. The day after the assay, as much hair as possible were removed from the animals by applying a layer of commercial hair-removing cream to remove all the trace of hair and to leave the skin of the back completely bare. The rest of the hair-removing cream were removed by means of washing and rinsing with running water and the animals were the returned to their corresponding cages where they remained until the following day.

On the day of the assay, the substances to be assayed were blindly applied on the back of the previously labelled animals following a random pattern (randomized). After applying the corresponding treatments, the animals were placed and pinned to the presentation platform of the equipment. The animals were then placed 8 cm from an ultraviolet light source of 4*40w with 313 nm of fundamental emission and a range comprised approximately between 290 and 350 nm and with an anti-UVC filter. The emission spectrum of said light source is depicted in Figure 1.

The exposure was maintained until all the animals have received an approximate total dose of 2.5 kJ/$m^2$. The dose of irradiation applied was determined by means of a ultraviolet light detector, the response curve of which is shown in Figure 2.

Twenty four hours after ending the exposure, the back of the animals was photographed and they were returned to their cages. The evaluation of erythema was performed by means of a positive/negative criterion, the percentage of protected animals being obtained. The statistic significance of the difference was evaluated by means of the non-parametric Fisher Test.

In order to determine the absorption spectrum, a solution of 10 mg/ml of methanol was prepared from each of the substances. The methanol in turn was used to adjust the zero absorbance in each of the wavelengths in the range of 195 - 400 nm.

The study was performed according to the guidelines of "Good Laboratory Practice".

## Results

After 24 hours of exposure, all the animals showed intense erythema accompanied by evident inflammatory signs which were very intense in some occasions and with variable hematic extravasation area which were ranged from small spots to clearly hemorrhagic lesions.

The result of the application of p-nitrolophine and m-nitrolophine was comparable. The fragments of skin on which these gels were applied had an almost normal appearance although a very light erythema could be seen. 100% of the animals were marked as "protected" because the intensity of the erythema was considerably reduced with respect to the surrounding areas. However, the suppression was not complete. The intensity of the erythema is between 60% and 80% lesser than the adjacent areas (see Figures 3 and 4 for compounds p-nitrolophine and m-nitrolophine, respectively).

The application of P5000 effectively protected 100% of the animals against erythema in which the skin had a rosy appearance very similar to the normal skin which can be subjectively evaluated in 70-90% of erythema suppression (see Figure 5).

Similarly, the treatment with P1789 visibly reduced the erythema in all the animals although the suppression achieved was considerably lesser than that obtained by p-nitrolophine, m-nitrolophine and by P5000. It was subjectively evaluated that the suppression achieved ranged between 10% and 30% with respect to the neighboring areas (see Figure 6).

The spectrum of 4-methylbenzylidene camphor (P5000) is characteristic as it has a maximum absorption between approximately 245 and 325 nm (295 nm), meanwhile the maximum absorption of butyl methoxydibenzoylmethane (P1789) is between approximately 305 and 395 nm (360 nm). This characteristic defines these substances as specific UVB and UVA filters, respectively.

The absorption spectrum of p-nitrolophine shows a maximum absorption in the UVA region comprised approximately between 320 and 450 nm (379 nm), although the UVB region also shows absorption. Therefore, p-nitrolophine offers an absorbance which simultaneously blocks the UVB and UVA light, even including the near visible wavelengths. Part of its effects could be due to a broad spectrum screen effect preventing the ultraviolet radiation from contacting the skin. By comparing the maximum obtained with the two patterns and p-nitrolophine, it was observed that, at the concentration used (10 mg/ml), the blocking of UVB by p-nitrolophine is lower than that of P5000 but higher than that of P1789, meanwhile in the UVA region the p-nitrolophine offers a very broad protection which also covers the near visible region (see Figure 7).

The absorption spectrum of m-nitrolophine shows a maximum absorption at wavelengths comprised approximately between 250 and 350 nm (309 nm). Therefore, the m-nitrolophine offers an absorbance which mainly blocks the UVB-UVA2 light and leaves the UVAL portion to pass almost intact. Part of its effects could be due to a screen effect on the UVB-UVA2 region. By comparing the maximums obtained with the two patterns and m-nitrolophine, it was

observed that, at the concentration used (10 mg/ml), the blocking of UVB by m-nitrolophine is higher than that of P5000, meanwhile in comparison with P1789 the blocking observed for m-nitrolophine is similar in the UVA2 region and very low in the UVA1 region. (see Figure 8).

Table 8 summarizes these results.

Table 8

| Erythema induced by UVB radiation in mouse | | | | |
|---|---|---|---|---|
| No. | p-nitrolophine | m-nitrotophine | P5000 | P1789 |
| 1 | - | - | - | +/- |
| 2 | - | - | - | +/- |
| 3 | - | - | - | +/- |
| 4 | - | - | - | +/- |
| 5 | - | - | - | -/+ |
| 6 | - | - | - | |
| 7 | - | - | - | |
| 8 | - | - | - | |
| 9 | - | - | - | |
| 10 | - | - | - | |
| % Protection | 100%* | 100%* | 100%* | 100%* |
| % Suppression | 60 - 80%* | 60 - 80%* | 70 - 90%* | 10-30% |
| * p<0.05 against non treated area | | | | |

Photoprotective effect of 0.5% p-nitrolophine, 0.5% m-nitrolophine, 0.5% 4-methylbenzylidene camphor (P5000) and 0.5% butyl methoxydibenzoylmethane (P1789). Results expressed as the presence (+) or absence (-) of erythema or its manifestations in the treatment area. The term "protected" refers to the number of animals which show no signs in the application area and if they are signs, these signs are of little importance (subjective criterion). The term "suppression" refers to the intensity of the erythematosus signs in the application area with relation to the surrounding area which was not treated (subjective criterion).

EXAMPLE 5

Evaluation of the topical anti-inflammatory property

The topical anti-inflammatory activity of p-nitrolophine has been analyzed in this study since erythema is a inflammatory process. Specifically, the inhibition of inflammation induced by phorbol 12-myristate-13-acetate (PMA) was evaluated and compared with an anti-inflammatory product of reference (indometacin). 37 mice were divided into the following groups: positive control group (PMA 0.5 mg); negative control group (indometacin 0.5 mg); treated groups (0.5 mg and 1 mg of p-nitrolophine). All the solutions were prepared using acetone (ppa) as vehicle. 20 $\mu$L of solution of 25 mg/mL of phorbol 12-myristate-13-acetate (PMA) [Fluka cod 79346] (10 $\mu$L in each face) was administered in the right ear of each mouse. Immediately after administering the PMA, nothing more was administered into the positive control group; 20 $\mu$L of the solution of 25 mg/mL of indometacin was administered to the negative control group, and 20 $\mu$L of the solution of 25 or 50 mg/mL of 4-nitrolophine was administered in the same manner to the treated groups. Animals having no lesions or skin and ear irritation were used.

After 4 hours of post-treatment, the mice were sacrificed through cervical fracture and a portion of 6 mm in diameter was obtained from each ear with a hollow punch which was weight in precision analytical balance. The difference in weight ($\Delta$) between the right ear (treated) and the left ear (non treated) for each animal and for each group were calculated. The percentage of inhibition (% inh) was estimated and calculated according to the following equation:

$$\% \text{ inh} = 100\ (1-\Delta t/\Delta c)$$

wherein Δt represents the difference in the treated groups and Δc the difference for the negative control group.

In order to analyze the results, an one way ANOVA and Dunnet test for multiple comparison was used, values of P<0.05 were considered significant. A software, Graphpad Instat Version 3.06, was used for such purposes.

The data of the weights of the animals and of the right and left ears are described in Table 9.

Table 9

| Group | Mouse No. | Weight | RE | LE |
|---|---|---|---|---|
| PMA 0.5 mg | 1 | 27.1 | 12 | 6,5 |
| | 2 | 30.1 | 10.4 | 7,9 |
| | 3 | 27 | 9.2 | 6.8 |
| | 4 | 27.2 | 12.6 | 8,2 |
| | 5 | 26.6 | 15.2 | 7,4 |
| | 6 | 27.2 | 12.9 | 6.3 |
| | 7 | 26.8 | 13.6 | 6 9 |
| | 8 | 25.5 | 12.9 | 6 4 |
| | 9 | 24.5 | 13.2 | 6,2 |
| | 10 | 26.8 | 10.8 | 5,8 |
| Average SD | | 26.9<br>1.4 | 12.3<br>1.7 | 6.8<br>0.8 |
| Indometacin 0.5 mg | 11 | 24.5 | 6.7 | 6.2 |
| | 12 | 25.8 | 7.2 | 6.4 |
| | 13 | 29.1 | 6.8 | 6.9 |
| | 14 | 27.1 | 7.2 | 6.7 |
| | 15 | 26.5 | 10 | 6.9 |
| | 16 | 26.4 | 6.2 | 7.6 |
| | 17 | 28.3 | 7.4 | 6.7 |
| | 18 | 27 | 9.1 | 6.7 |
| | 19 | 25.4 | 6.8 | 5.5 |
| | 20 | 26 | 6.2 | 7.2 |
| Average SD | | 26.6<br>1,4 | 7.4<br>1.2 | 6.7<br>0.6 |
| 4-nitrolophine 0.5 mg | 21 | 29.5 | 7.3 | 9.9 |
| | 22 | 30.6 | 7 | 7.5 |
| | 23 | 28.4 | 5.9 | 6.2 |
| | 24 | 29.4 | 5.9 | 6.4 |
| | 25 | 30.1 | 5.6 | 6.7 |
| | 26 | 26.6 | 6.6 | 6.7 |
| | 27 | 28.5 | 7.2 | 7.5 |
| | 28 | 26.9 | 7.8 | 5.6 |
| | 29 | 27.4 | 6.5 | 6.2 |
| | 30 | 26.4 | 6 | 5.2 |
| | 31 | 25 | 6.6 | 5.9 |

(continued)

| Group | Mouse No. | Weight | RE | LE |
|---|---|---|---|---|
| | 32 | 29.1 | 7.7 | 7.8 |
| | 33 | 28.2 | 5.8 | 6 |
| Average | | 28.2 | 6.6 | 6.7 |
| SD | | 1.6 | 0.7 | 1.2 |
| 4-Nitrolophine 1 mg | 34 | 26 8 | 5.9 | 5.6 |
| | 35 | 29.4 | 7.3 | 5.8 |
| | 36 | 24.6 | 6.2 | 5,5 |
| | 37 | 29.1 | 6.2 | 6.6 |
| Average | | 27.5 | 6.4 | 5.9 |
| SD | | 2.2 | 0.6 | 0.5 |

All the animals had normal behavior throughout the study period. The results disclose the difference in weight between the right and left ear ($\Delta$ = RE - LE) for each mouse and for each group in accordance to the methodology described. Table 10 and Figure 10 shows the average and SEM of ($\Delta$ = RE - LE) for each group (values of $p < 0.05$ were considered significant).

Table 10

| Group | Control (A) | Indometacin (B) | 4-Nitrolophine (C) | 4-Nitrolophine (D) |
|---|---|---|---|---|
| Dose (mg/ear) | - | 0.5 | 0.5 | 1 |
| Average | 5.44 | 0.61 | -0.13 | 0.52 |
| SEM (±) | 0.59 | 0.43 | 0.30 | 0.40 |
| 95% conf. limit (lower/higher) | (4.11/6.77) | (0.31/1.65) | (-0.8/0.53) | (-0.73/1.79) |
| Sample size (n) | 10 | 10 | 13 | 4 |
| % inhibition | - | 87.7** (q = 7.6) | 100** (q = 9.5) | Too little data for statistics |
| ** P< 0.01 (q> 2.47) | | | | |

The group D had few animals (n=4) therefore, although it was not statistically compared a tendency to inhibit inflammation (90% approximately) was observed. When comparing the treatment with indometacin (Group B) with that of 4-nitrolophine (C) at the same dose (0.5 mg), significant difference were not observed (q = 1.3, p > 0.05).

Therefore, by using this preliminary assay it can concluded that 4-nitrolophine at the dose of 0.5 mg inhibits inflammation induced by PMA.

The aforementioned examples proved that the compounds of formula (I) of the present invention provide a double protection mechanism:

- on one hand, they are useful for blocking the access of ultraviolet and near visible radiation to the surface of the skin; and

- in the other, they are useful for reducing the inflammation produced by those radiations which managed to reach the skin.

**Claims**

1. A composition comprising (i) a compound A that absorbs in the near visible-ultraviolet A (UVA) region and (ii) a compound B that absorbs in the ultraviolet B (UVB) region, wherein at least one of said compounds A or B is a

compound of formula (I)

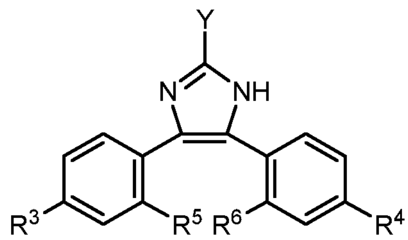

(I)

wherein
Y represents

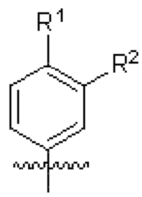

or

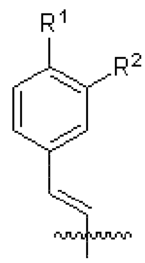

$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and
$R^5$ and $R^6$ independently of each other represent H, or alternatively $R^5$ and $R^6$ together form a C-C bond between the carbon atoms to which they are bound.

**2.** The composition according to claim 1, wherein said compound of formula (I) is selected from the group consisting of:

2,4,5-triphenyl-1H-imidazole;
2-(4-hydroxyphenyl)-4,5-diphenyl-1H-imidazole;
2-(4-methoxyphenyl)-4,5-diphenyl-1H-imidazole;
2-(3-nitrophenyl)-4,5-diphenyl-1H-imidazole;
2-(4-nitrophenyl)-4,5-diphenyl-1H-imidazole;
2-(4-chlorophenyl)-4,5-diphenyl-1H-imidazole;
2-(4-fluorophenyl)-4,5-diphenyl-1H-imidazole;
2-[4-(N,N-dimethylamino)phenyl]-4,5-diphenyl-1H-imidazole;
(E)-2-styryl-4,5-diphenyl-1H-imidazole;
(E)-2-(4-nitrostyryl)-4,5-diphenyl-1H-imidazole;
2,4,5-tris(4-nitrophenyl)-1H-imidazole; and
2-(4-nitrophenyl)-1H-phenanthro[9,10-d]imidazole.

**3.** The composition according to any of claims 1 to 2, comprising two or more compounds of formula (I) preferably selected from the group defined in claim 2 and mixtures thereof.

**4.** The composition according to any of claims 1 to 3, comprising

(i) a compound of formula (I) selected from 2-(4-nitrophenyl)-4,5-diphenyl-1H-imidazole, 2-[4-(N,N-dimethylamino)phenyl]-4,5-diphenyl-1H-imidazole, (E)-2-styryl-4,5-diphenyl-1H-imidazole, (E)-2-(4-nitrostyryl)-4,5-diphenyl-1H-imidazole, 2,4,5-tris(4-nitrophenyl)-1H-imidazole, 2-(4-nitrophenyl)-1H-phenanthro[9,10-d]imidazole, and mixtures thereof; and
(ii) a compound of general formula (I) selected from 2,4,5-triphenyl-1H-imidazole, 2-(4-hydroxyphenyl)-4,5-diphenyl-1H-imidazole, 2-(4-methoxyphenyl)-4,5-diphenyl-1H-imidazole, 2-(3-nitrophenyl)-4,5-diphenyl-1H-imidazole, 2-(4-chlorophenyl)-4,5-diphenyl-1H-imidazole, 2-(4-fluorophenyl)-4,5-diphenyl-1H-imidazole, and

mixtures thereof.

**5.** The composition according to any of claims 1 to 4, further comprising a complementary solar filter.

**6.** The composition according to any of claims 1 to 5 in a topical dosage form.

**7.** A compound of formula (I'')

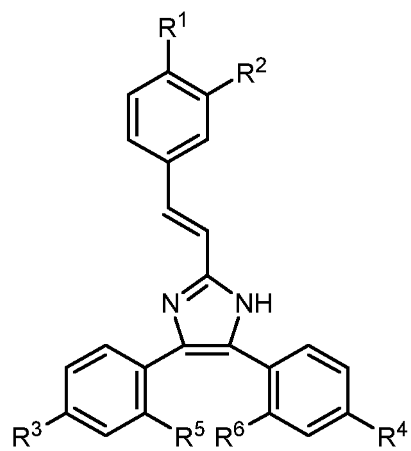

(I'')

wherein
$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and
$R^5$ and $R^6$ independently of each other represent H, or alternatively $R^5$ and $R^6$ together form a C-C bond between the carbon atoms to which they are bound.

**8.** The compound according to claim 7, wherein $R^1$, $R^2$, $R^3$ and $R^4$ independently of each other represent H, OH, OMe, $NH_2$, NHMe, $NMe_2$, $NO_2$, fluorine or chlorine; preferably the compound is (E)-2-styryl-4,5-diphenyl-1H-imidazole or (E)-2-(4-nitrostyryl)-4,5-diphenyl-1H-imidazole.

**9.** Use of a compound of formula (I)

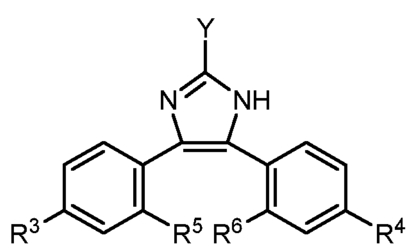

(I)

wherein
Y represents

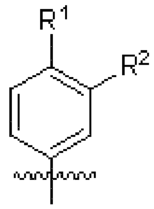

or

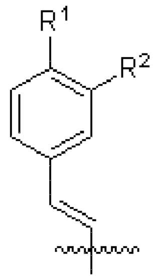

$R^1$, $R^2$, $R^3$ and $R^4$ independently of each other represent H, OH, $OR_a$, $NH_2$, $NHR_a$, $NR_aR_b$, $NO_2$ or halogen; wherein $R_a$ and $R_b$ independently of each other represent $C_1$-$C_{10}$ alkyl; and
$R^5$ and $R^6$ independently of each other represent H, or alternatively $R^5$ and $R^6$ together form a C-C bond between the carbon atoms to which they are bound,
in the preparation of a photoprotective composition.

**10.** Use according to claim 9, wherein said compound of formula (I) is selected from the group consisting of:

2,4,5-triphenyl-1H-imidazole;
2-(4-hydroxyphenyl)-4,5-diphenyl-1H-imidazole;
2-(4-methoxyphenyl)-4,5-diphenyl-1H-imidazole;
2-(3-nitrophenyl)-4,5-diphenyl-1H-imidazole;
2-(4-nitrophenyl)-4,5-diphenyl-1H-imidazole;
2-(4-chlorophenyl)-4,5-diphenyl-1H-imidazole;
2-(4-fluorophenyl)-4,5-diphenyl-1H-imidazole;
2-[4-(N,N-dimethylamino)phenyl]-4,5-diphenyl-1H-imidazole;
(E)-2-styryl-4,5-diphenyl-1H-imidazole;
(E)-2-(4-nitrostyryl)-4,5-diphenyl-1H-imidazole;
2,4,5-tris(4-nitrophenyl)-1H-imidazole; and
2-(4-nitrophenyl)-1H-phenanthro[9,10-d]imidazole.

**11.** Use according to any of claims 9 or 10 of said photoprotective composition for protecting skin or hair from solar radiation or from other radiation of a natural or artificial origin.

**12.** Use according to claim 11 of said photoprotective composition for protecting skin or hair from A-type, B-type and/or near visible-type ultraviolet radiation.

**13.** Use according to any of claims 11 or 12 of said photoprotective composition for preventing or minimizing the damaging effects produced by said radiation on the skin or hair, wherein said damaging effects are preferably selected from erythema, severe sunburns, sun-induced premature aging of the skin, regulation of biochemical deterioration caused by free radicals, photocarcinogenesis, melanoma and non-melanoma skin cancer.

**14.** Use according to claim 13 of said photoprotective composition for preventing or minimizing the damaging effects produced by said radiation on the skin or hair in subjects at risk of hormone disruption.

**15.** A cosmetic treatment method for protecting skin from solar radiation or other radiation of a natural or artificial origin or for preventing or minimizing the damaging effects produced by solar radiation or other radiation of a natural or artificial origin on the skin or hair, which comprises applying on the skin or hair an effective amount of a composition as defined in any one of claims 1 to 8.

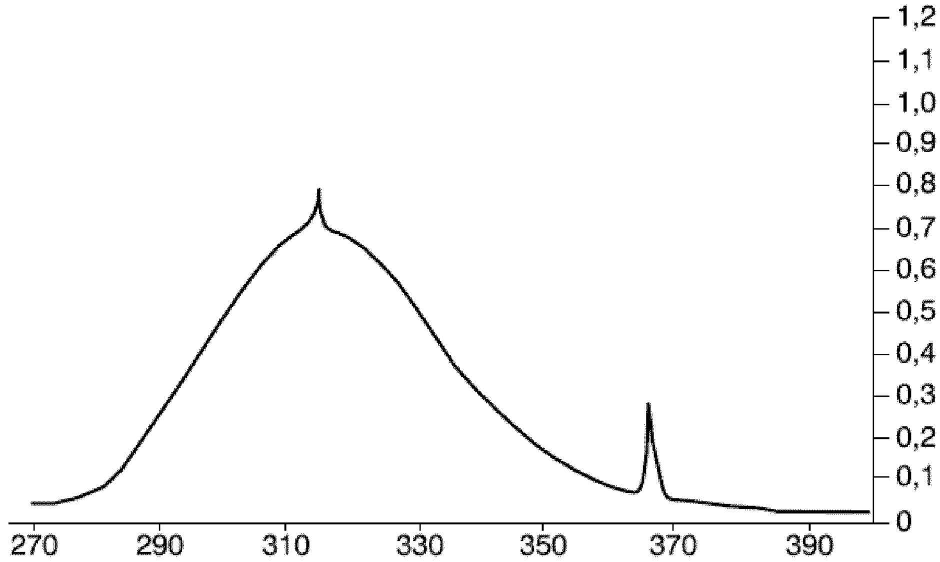
1,2
1,1
1,0
0,9
0,8
0,7
0,6
0,5
0,4
0,3
0,2
0,1
0
270
290
310
330
350
370
390

FIG. 1

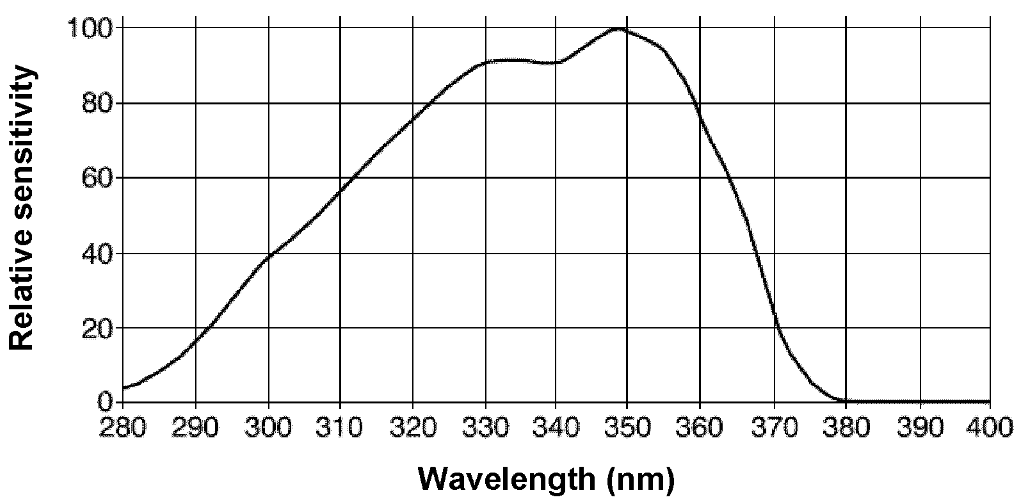
Relative sensitivity
100
80
60
40
20
0
280
290
300
310
320
330
340
350
360
370
380
390
400
Wavelength (nm)

FIG. 2

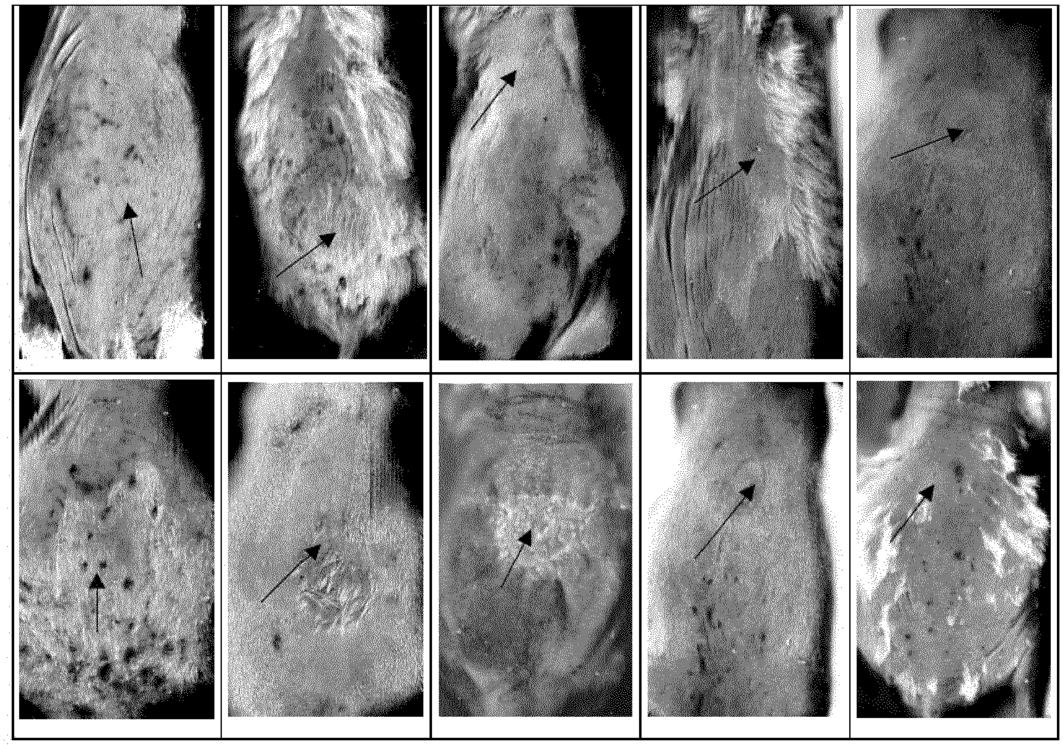

FIG. 3

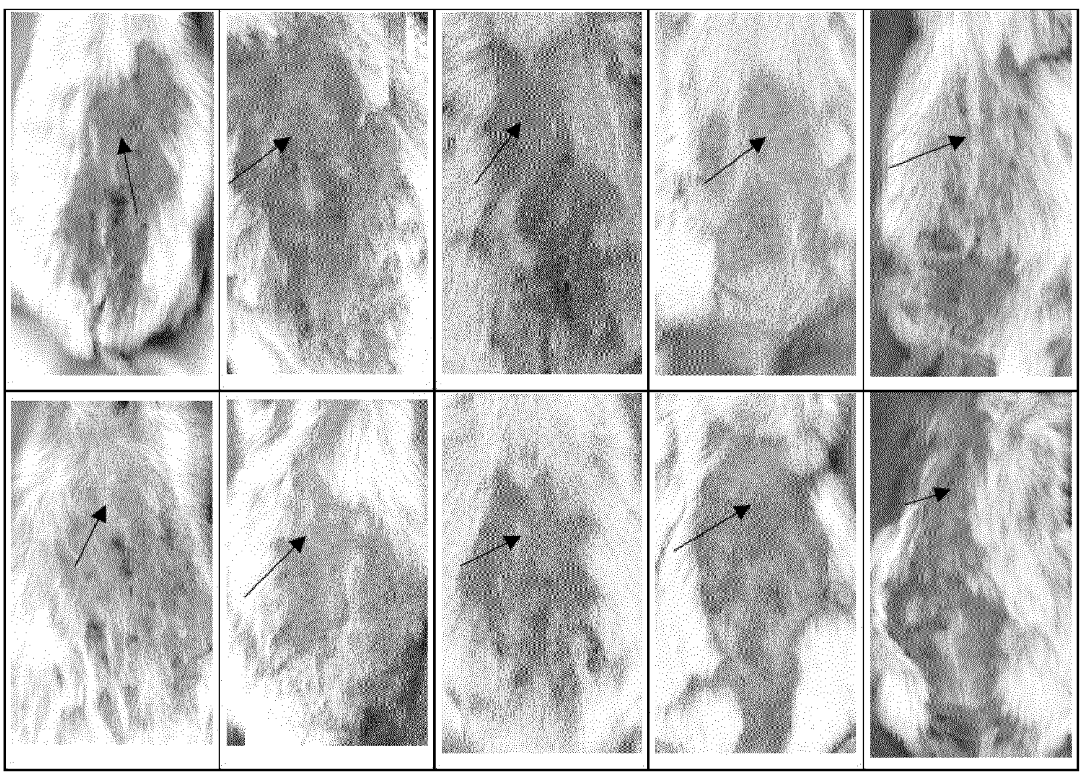

FIG. 4

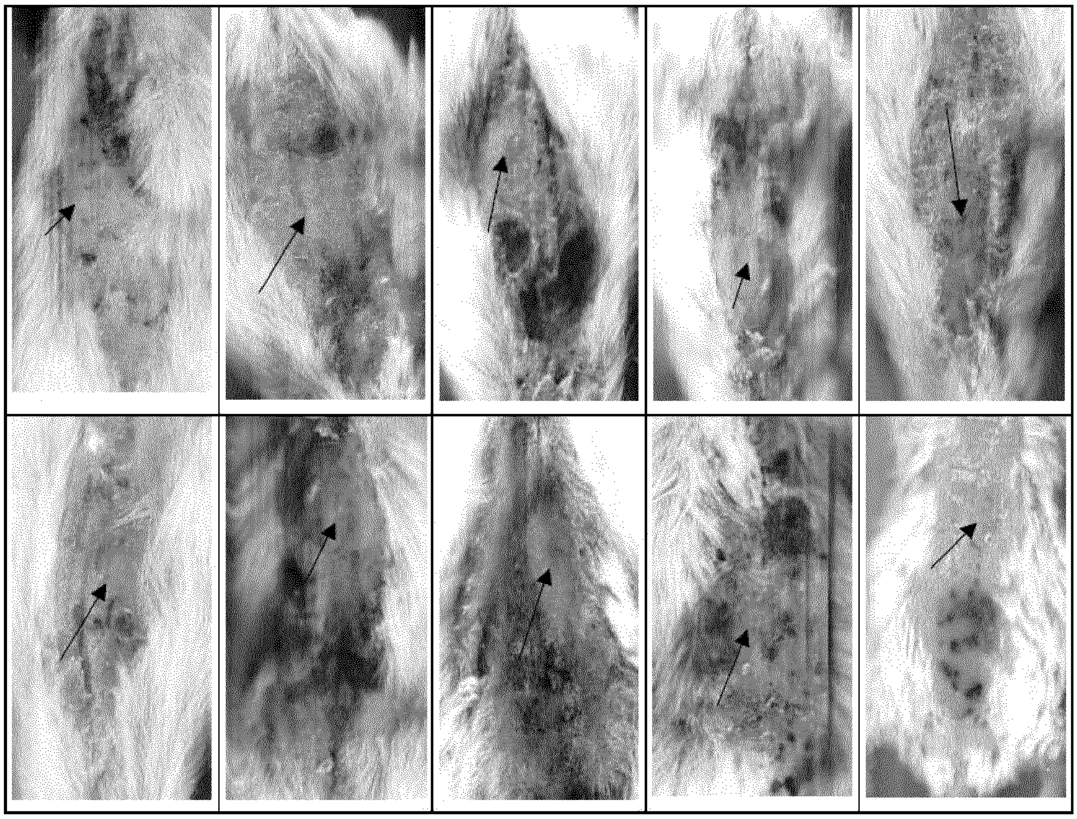

FIG. 5

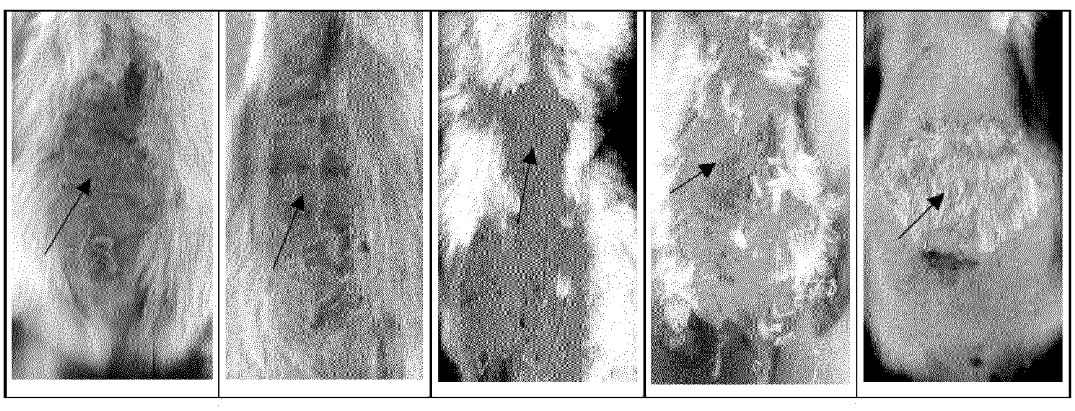

FIG. 6

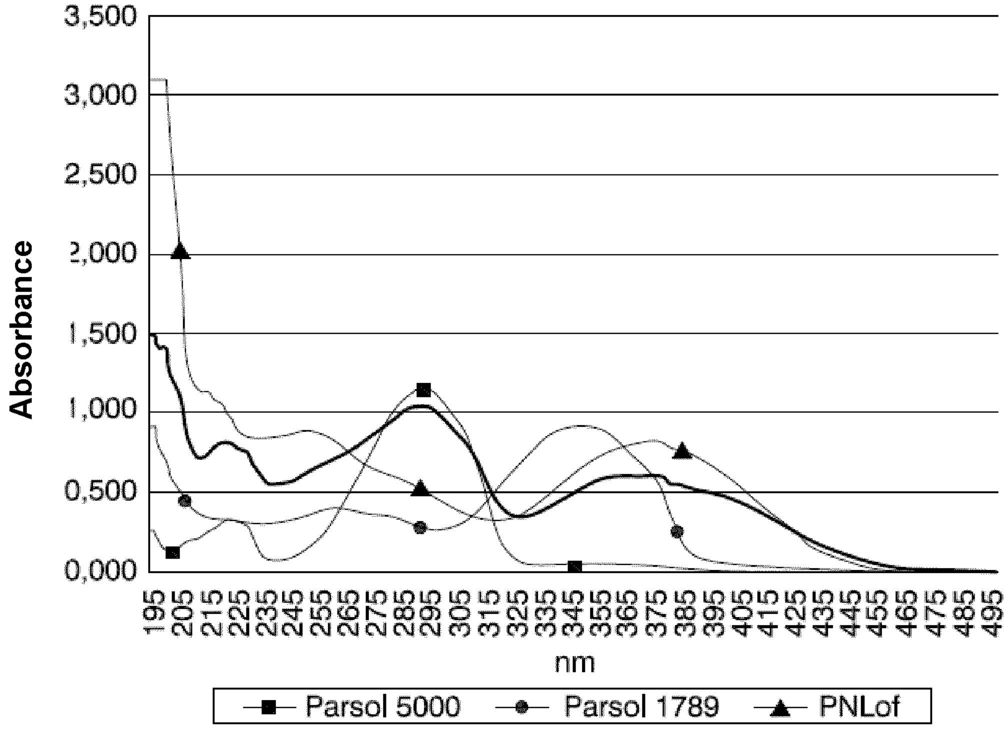
3,500
3,000
2,500
2,000
1,500
1,000
0,500
0,000
Absorbance
195 205 215 225 235 245 255 265 275 285 295 305 315 325 335 345 355 365 375 385 395 405 415 425 435 445 455 465 475 485 495
nm
Parsol 5000
Parsol 1789
PNLof

FIG. 7

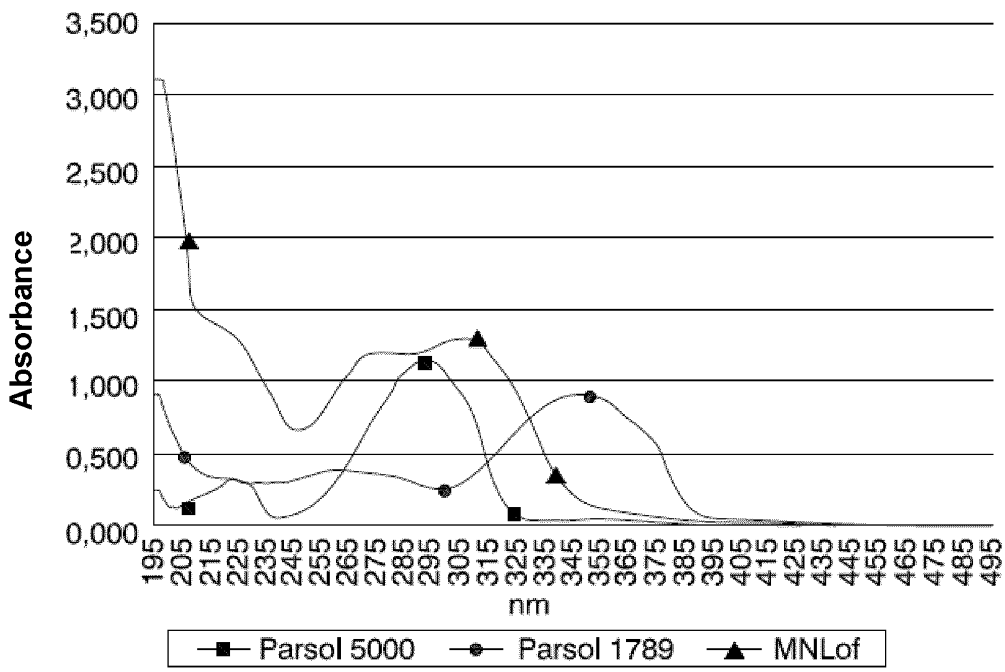
3,500
3,000
2,500
2,000
1,500
1,000
0,500
0,000
Absorbance
195 205 215 225 235 245 255 265 275 285 295 305 315 325 335 345 355 365 375 385 395 405 415 425 435 445 455 465 475 485 495
nm
Parsol 5000
Parsol 1789
MNLof

FIG. 8

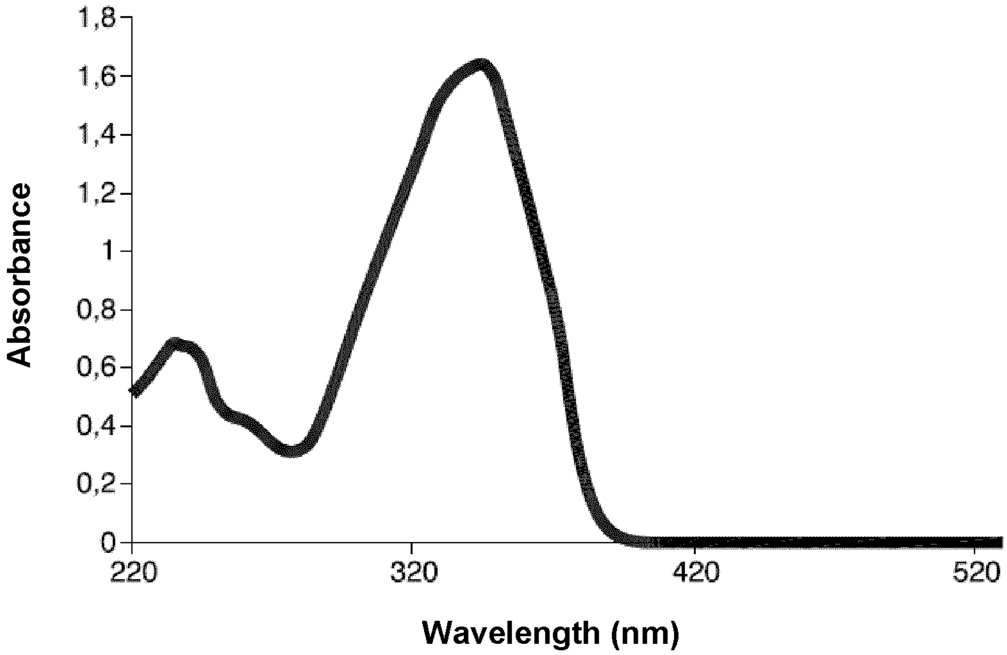
1,8
1,6
1,4
1,2
1
0,8
0,6
0,4
0,2
0
Absorbance
220
320
420
520
Wavelength (nm)

FIG. 9

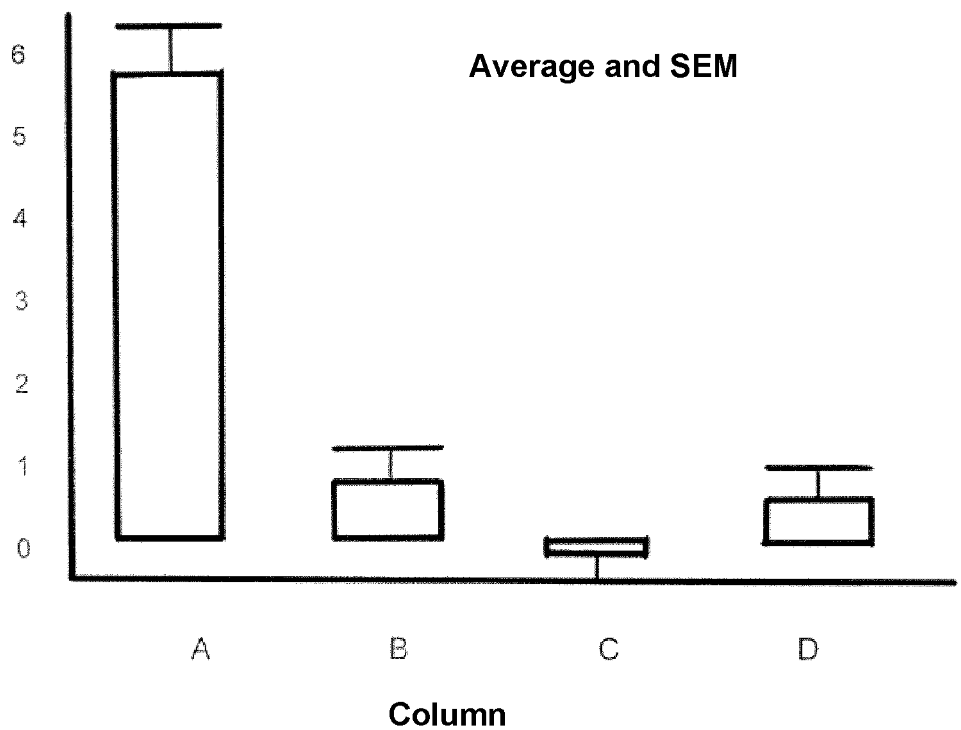


FIG. 10

## INTERNATIONAL SEARCH REPORT

International application No.
PCT/ ES 2010/070397

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)
C07D, A61K, A61Q

Documentation searched other than minimum documentation to the extent that such documents are included in the fields searched

Electronic data base consulted during the international search (name of data base and, where practicable, search terms used)

INVENES, EPODOC, WPI, NPL, XPESP, MEDLINE, BIOSIS, REGISTRY, HCAPLUS.

C. DOCUMENTS CONSIDERED TO BE RELEVANT

| Category* | Citation of document, with indication, where appropriate, of the relevant passages | Relevant to claim No. |
|---|---|---|
| X | MATSUDA, I. et al. "Cyclization reactions by the use of 1,2-bis(trimethylsilyl)imino-1,2-diphenylethane". Chemistry letters, 1977, pages 1457-1460. See page 1458, paragraph 1, scheme 1, compound 3; page 1459, table, example 9. | 7,8 |
| X | JP 63239274 A (TOYAMA CHEM CO LTD) 05.10.1988. See abstract, World Patent Index [online]. London (Reino Unido) Thompson Publications, Ltd. [retrieved on 19.08.2010] DW198846, Accession Number 1988-326017. | 7,8 |
| X | US 2005/0118123 A1 (VAIDYA, N. A.) 02.06.2005. See page 3, claims 1 and 3. | 9-14 |
| X | KR 20080103283 A (LG HOUSEHOLD & HEALTH CARE LTD) 27.11.2008. See abstract, EPODOC/EPO [retrieved on 19/08/2010]. | 9-14 |

☒ Further documents are listed in the continuation of Box C. ☒ See patent family annex.

* Special categories of cited documents:

"A" document defining the general state of the art which is not considered to be of particular relevance.

"E" earlier document but published on or after the international filing date

"L" document which may throw doubts on priority claim(s) or which is cited to establish the publication date of another citation or other special reason (as specified)

"O" document referring to an oral disclosure use, exhibition, or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other documents , such combination being obvious to a person skilled in the art

"&" document member of the same patent family

| Date of the actual completion of the international search | Date of mailing of the international search report |
|---|---|
| 30 September 2010 (30.09.2010) | **(08.09.2010)** |
| Name and mailing address of the ISA/<br>O.E.P.M.<br>Paseo de la Castellana, 75 28071 Madrid, España.<br>Facsimile No. 34 91 3495304 | Authorized officer<br>N. Martín Laso<br>Telephone No. +34 91 349 32 78 |

Form PCT/ISA/210 (second sheet) (July 2009)

**INTERNATIONAL SEARCH REPORT**

International application No.
PCT/ES 2010/070397

C (continuation). DOCUMENTS CONSIDERED TO BE RELEVANT

| Category* | Citation of documents, with indication, where appropriate, of the relevant passages | Relevant to claim No. |
|---|---|---|
| A | US 2003/0103915 A1 (QUINTINE, M.) 05.06.2003. See paragraphs 1, 48 and 57. | 1-15 |
| A | DE 19849514 A1 (BUNDESREP DEUTSCHLAND) 04.05.2000. See abstract, EPODOC/EPO [recuperado 19/08/2010]. | 1-15 |

Form PCT/ISA/210 (continuation of second sheet) (July 2009)

**INTERNATIONAL SEARCH REPORT**

Information on patent family members

International application No.

PCT/ ES 2010/070397

| Patent document cited in the search report | Publication date | Patent family member(s) | Publication date |
|---|---|---|---|
| JP 63239274 A | 05.10.1988 | JP 8030063 B | 27.03.1996 |
| US 2005118123 A | 02.06.2005 | NONE | ------------ |
| KR 20080103283 A | 27.11.2008 | KR 100871911 B | 05.12.2008 |
| US 2003103915 A | 05.06.2003 | ITMI 20012037<br>EP 1300137 A | 02.04.2003<br>09.04.2003 |
| DE 19849514 A C | 04.05.2000 | NONE | ------------ |

Form PCT/ISA/210 (patent family annex) (July 2009)

**INTERNATIONAL SEARCH REPORT**

International application No.

PCT/ ES 2010/070397

CLASSIFICATION OF SUBJECT MATTER

***C07D 233/64*** (2006.01)
***A61K 8/49*** (2006.01)
***A61Q 17/04*** (2006.01)

Form PCT/ISA/210 (extra sheeet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4387089 A
- US 4859585 A

### Non-patent literature cited in the description


- Sunscreen and Suntan Products. **DeSimone.** Handbook of Nonprescription Drugs. American Pharmaceutical Association, 1986, 533-546
- **Grove ; Forbes.** A Method for Evaluating the Photoprotection Action of Sunscreen Agents Against UV-A Radiation. *International Journal of Cosmetic Science,* 1982, vol. 4, 15-24
- **De Méo et al.** Genotoxicity of visible light (400-800 nm) and photoprotection assessment of ectoin, L-ergothioneine and mannitol and four sunscreens. *Journal of Photochemistry and Photobiology B: Biology,* 2008, vol. 91, 24-34
- **Parrish JA et al.** The Optics of Human Skin. *The Journal of Investigative Dermatology,* 1981, vol. 77, 13-19
- **Pathak M A.** Sunscreens: Progress and perspectives on photoprotection of human skin against UVB and UVA radiation. *J Dermatol,* 1996, vol. 23 (11), 783-800
- **Gilaberte Y ; Coscojuela C ; Saenz de Santamaria M C ; Gonzalez S.** Photoprotectores. *Actas Dermosifiliogr,* 2003, vol. 94 (5), 271-293
- **Philbrook, G E.** *Photochemisty and Photobiology,* 1965, vol. 4, 1175-1183
- **Crouch et al.** Microwave-Mediated Synthesis of Lophine: Developing a Mechanism to Explain a Product. *Journal of Chemical Education,* 2006, vol. 83, 1658-1660
- **N. Shaath et al.** Sunscreens: Development, Evaluation and Regulatory Aspects. Marcel Dekker, Inc, 1997, 269-273
- **S. Nakada ; H. Konishi.** Sun Protection Effect of Nonorganic Materials. *Fragrance Journal,* 1987, vol. 15, 64-70
- CTFA Cosmetic Ingredient Handbook. 1997
- CTFA COSMETIC INGREDIENT HANDBOOK. 2000
- An in culture bioassay to assess the estrogenicity of xenobiotics. **Soto et al.** Chemically Induced Alterations in Sexual Development: The Wildlife/Human Connection. Princeton Scientific Publishing, 1992, 295-309
- **Soule et al.** A human cell line from a pleural effusion derived from a breast carcinoma. *J. Natl. Cancer Inst.,* 1973, vol. 51, 1400-1413
- **Soto et al.** The E-Screen as a tool to identify estrogens: an update on oestrogenic environment pollutants. *Environ. Health Perspect.,* 1995, vol. 103, 113-122
- **Villalobos et al.** The E-SCREEN assay: comparison among different MCF7 cell stocks, Environ. *Health Perspect.,* 1995, vol. 103, 844-850
- **Winder et al.** A study of Pharmacological influences on ultraviolet erythema in guinea pigs. *Arch. Int Pharmacodyn,* 1958, vol. 116, 261-292
- **Wendy et al.** The local antinociceptive and topical antiinflamatory effects of propyl gallate in rodents. *Br. J. Pharmacol,* 1976, vol. 58, 573-581
- **Katiyar et al.** Protective Effects of Silymarin Against Photocarcinogenesis in a Mouse Skin Model. *J Natl Cancer Inst,* 1997, vol. 89, 556-65